(19) Europäisches Patentamt
European Patent Office
Office européen des brevets

(11) **EP 2 415 782 A1**

(12) **EUROPEAN PATENT APPLICATION**
published in accordance with Art. 153(4) EPC

(43) Date of publication:
08.02.2012 Bulletin 2012/06

(21) Application number: **10758101.9**

(22) Date of filing: **30.03.2010**

(51) Int Cl.:
*C07K 14/395* (2006.01)      *A61K 36/064* (2006.01)
*A61K 39/395* (2006.01)      *A61P 31/10* (2006.01)
*A61P 35/04* (2006.01)

(86) International application number:
**PCT/ES2010/070196**

(87) International publication number:
**WO 2010/112656 (07.10.2010 Gazette 2010/40)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **02.04.2009 ES 200900908**

(71) Applicant: **Universidad Del País Vasco 48940 Leioa (Vizkaia) (ES)**

(72) Inventors:
• **HERNANDO ECHEVARRÍA, Fernado**
  **E-48940 Leioa (Vizkaia) (ES)**
• **RAMÍREZ GARCÍA, Andoni**
  **E-48940 Leioa (Vizkaia) (ES)**

(74) Representative: **ABG Patentes, S.L.**
  **Avenida de Burgos 16D**
  **Edificio Euromor**
  **28036 Madrid (ES)**

(54) **COMPOUNDS STIMULATING UNDESIRABLE CELLULAR ADHESION AND APPLICATIONS THEREOF**

(57)    The invention relates to non-glycosylated proteins, based on the Kre9 protein of *Candida albicans,* capable of stimulating cell adhesion and the use thereof in the identification of compounds potentially useful against diseases associated with unwanted cell adhesion. The invention also relates to the use of said non-glycosylated proteins in the treatment of diseases caused by infectious agents.

**EP 2 415 782 A1**

## Description

<u>Field of the Invention</u>

The present invention generally relates to the identification of non-glycosylated proteins capable of stimulating cell adhesion and the use thereof in the identification of compounds potentially useful against diseases associated with unwanted cell adhesion. The invention also relates to the use of said non-glycosylated proteins in the treatment of diseases caused by infectious agents.

<u>Background of the Invention</u>

Metastasis is the cellular mechanism whereby cells detached from a malignant tumor are displaced from their site of generation to another site without direct anatomic connection, wherein they reimplant. Their production involves a complex process comprising a succession of interdependent stages, in which the tumor cells colonize other organic territories after being spread through internal cavities or the lymphatic vascular and/or blood stream. Once metastasis is established, the invaded territory is parasitized, wherein various damaging effects from mechanical to endocrine effects are produced.

The most commonly accepted stages for explaining the metastatic process are:

a) <u>detachment and mobilization of the tumor cells</u>; depending on the location of the primary tumor, the tumor cells have a different ability of detaching from and leaving the tumor focus; the detached cells must then reach the blood or lymphatic vessels so they can be spread towards other distant organs;
b) <u>spreading of the tumor cells</u>; the cells that reach the blood stream, in addition to having to adapt to the different environmental conditions of the blood and the mechanical effects caused by circulatory hemodynamics, have to escape from the tumoricidal effect of the immune system;
c) <u>implantation of the metastatic cells in distant organs</u>; in this stage of the metastatic process three consecutive phenomenon have been described: the circulating tumor cells stop in the capillary lumen, they extravasate into the surrounding tissue and proliferate until metastasis is formed; and
d) <u>clonogenic proliferation and formation of metastasis</u>; once extravasated, the proliferation of the metastatic cells is different from that observed in the primary tumor, the tumor cells have an autoregulation conditioned by their own growth factors and oncogenes.

The existence of receptors for molecules of the extracellular matrix on the cell surface could be of vital importance for assuring the perfect fixing and subsequent proliferation of the tumor cell. The interaction between the circulating tumor cells and the endothelial cells is one of the processes which determines that the metastasis is organ-specific. Adhesion molecules that are expressed by both the tumor cell and the endothelial cell are involved in the adhesion process between the tumor and the vascular endothelium. This fact suggests that the metastatic potential of each tumor cell can be influenced by the type of adhesion molecules that are expressed during the interaction between the cancerous cell and the vascular endothelium, hence the identification of these molecules is considered as one of the fundamental objectives for the treatment and prevention of tumor spread.

Although the specific adhesion of the tumor cells to the microvascular endothelium is a proven fact, the involvement thereof in the development of the metastasis is still not known. It has been suggested that the cells adhered to the endothelium could be more protected from the attack of the circulating cytotoxic cells and consequently have more possibilities of prolonging their half life than staying in the blood stream. The tumor adhesion to the endothelium can also facilitate the cell motility necessary for extravasation thereof and contribute to another stage of the metastatic process, the tumor invasion of the parenchyma. It has also been proposed that during the metastatic process, the main value of the tumor adhesion to the endothelium corresponds to the transduction of signals from the latter to the viable tumor cells, which would serve as stimulus for the intravascular proliferation in the detention sites. In lungs, it has been observed that the proliferation of the tumor cells adhering to the endothelium causes the formation of intravascular micrometastases which increase in size thus breaking the vascular wall without the need of extravasation. Various morphological studies of the tumor adhesion to the endothelium have indicated that the adhesion takes place preferably in the binding area between neighboring endothelial cells. It has been observed that this process continues with an endothelial retraction and the rechanneling of the blood vessel around the tumor cells. Endothelial retraction leads to basal membrane exposure and promotes tumor adhesion to the extracellular matrix. A simultaneous stimulation of the synthesis and secretion of proteolytic enzymes by the endothelial cells could, in turn, favor this process. In this sense, it was demonstrated that the endothelium damaged by the action of oxygen free radicals releases active collagenases capable of degenerating the basal membrane and facilitating tumor invasion. Finally, in relation to the functional implications of tumor adhesion to the endothelium, the cytotoxic role of the endothelial cells on the tumor cells must be noted.

This cytotoxicity requires a direct contact between the tumor cells and the endothelium.

The prevention and the therapeutic control of metastasis is very difficult. The main reason for the medical failure lies in the non-existence of accurate information about its pathogenesis which entails a lack of specific resources for its prophylaxis or inhibition.

There is therefore a need to develop alternatives to those existing for preventing and controlling the metastasis which contribute to increase the arsenal of therapeutic remedies against said pathological situation.

Summary of the Invention

The inventors of the present invention have found that the non-glycosylated *C. albicans* Kre9 protein is surprisingly involved in the increase of the adhesion of melanoma tumor cells to the hepatic sinusoidal endothelium, thus inducing a metastatic process in the liver. Based on this fact, the inventors have opened up a new therapeutic window for the prevention and/or treatment of diseases associated with unwanted cell adhesion, such as tumor processes, metastatic processes, diseases that cause a cellular immune response, or inflammatory processes, particularly, processes mediated by cytokines, for example. Likewise, said protein can be used in the prevention and/or treatment of diseases caused by infectious agents due to its high proinflammatory potential.

Therefore, in one aspect, the invention relates to a non-glycosylated protein comprising the amino acid sequence SEQ ID NO: 1, or a variant or fragment thereof, capable of stimulating tumor adhesion to the endothelium, and to its use in the identification of compounds potentially useful against diseases associated with unwanted cell adhesion and in the treatment of said diseases.

In addition, in another aspect, the invention relates to the use of said non-glycosylated protein comprising the amino acid sequence SEQ ID NO: 1, or a functionally equivalent variant or fragment thereof, as an antigen in the prevention and/or treatment of a disease caused by an infectious agent, e.g., candidiasis.

The invention also relates to the use of a glycosylated protein capable of stimulating tumor adhesion to the endothelium, comprising the amino acid sequence SEQ ID NO: 1, or a variant or fragment thereof which maintains said stimulating ability to stimulate tumor adhesion to the endothelium, in the identification of compounds potentially useful against diseases associated with unwanted cell adhesion.

Brief Description of the Drawings

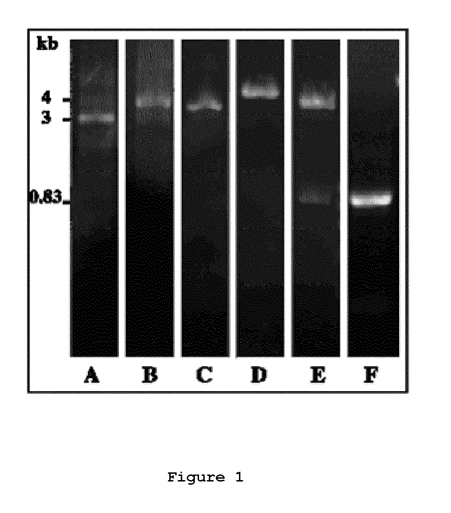

Figure 1 shows A: pET-Blue circular plasmid; B: pET-Blue linear plasmid, digested with one of the restriction enzymes; C: pET-KRE9 circular plasmid; D: pET-KRE9 linear plasmid digested with one of the restriction enzymes; E: pET-KRE9 plasmid digested with both restriction enzymes; and F: amplification product of the KRE9 gene by means of PCR. See Example 1 for additional information.

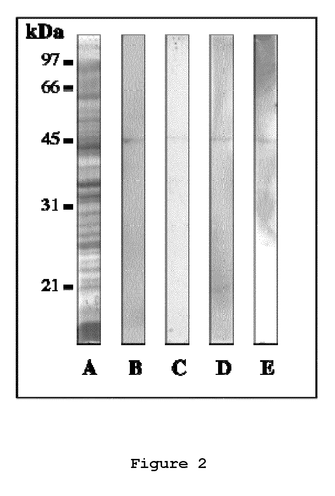

Figure 2 shows the results of A: SDS-PAGE electrophoresis and Coomassie blue staining of the *E.coli* protein extract after the expression of the CaKre9 protein is induced before purification; B: Western Blot of the *E.coli* extract + CaKre9 before being purified evaluated against the anti-HSV antibody; C: SDS-PAGE electrophoresis and Coomassie blue staining of the *E. coli* protein extract after the expression of the CaKre9 protein is induced after being purified; D: Western Blot of the recombinant CaKre9 protein after being purified evaluated against the anti-HSV antibody; and E: Western Blot of the recombinant CaKre9 protein after being purified evaluated against the anti-IL-1β antibody.

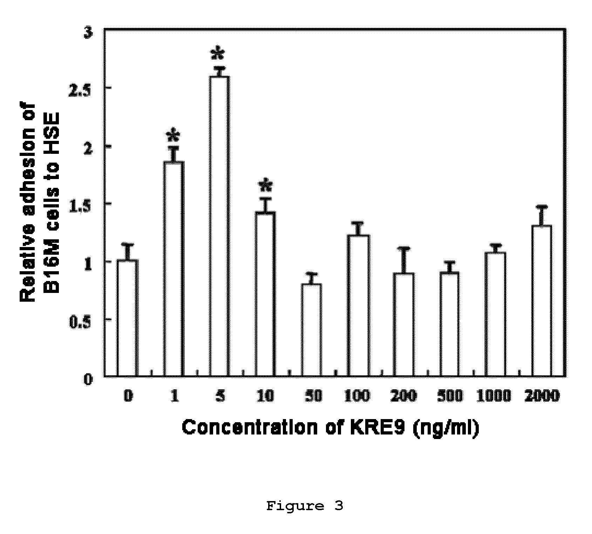

Figure 3 is a bar diagram in which the effect of the CaKre9-(His)6 fusion protein on the increase of tumor adhesion to the HSE can be seen. The differences are expressed as relative adhesion of the B16M cells to the HSE. The asterisk (*) indicates that the differences are significant.

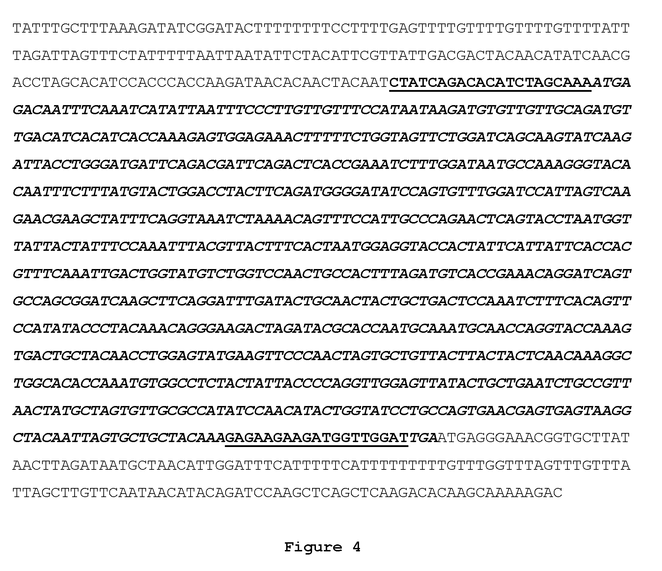

Figure 4 shows the sequence of the KRE9 gene of *C. albicans* (bold italic) and of the sequences used as primers (underlined). Said sequence is included in the Sequence listing identified as SEQ ID NO: 5.

Detailed Description of the Invention

I. Non-glycosylated protein of the invention, obtaining it and applications thereof

In one aspect, the invention relates to a non-glycosylated protein comprising the amino acid sequence shown in SEQ ID NO: 1, or a functionally equivalent variant or fragment thereof, capable of increasing the adhesion of tumor cells to the endothelium.

For the sake of simplicity, the expression "non-glycosylated protein of the invention" includes said non-glycosylated protein comprising the amino acid sequence identified as SEQ ID NO: 1, as well as the functionally equivalent variants and fragments thereof, capable of increasing the adhesion of tumor cells to the endothelium.

In a particular embodiment, the non-glycosylated protein of the invention comprises or consists of the amino acid sequence shown in SEQ ID NO: 1; said protein at least has the ability to increase the adhesion of tumor cells to the endothelium, an ability that can be determined by contacting tumor cells with endothelial cells stimulated with said non-glycosylated protein of the invention (see, for example, Example 2).

In a particular embodiment, the non-glycosylated protein of the invention is the non-glycosylated KRE9 protein of a species from the genus *Candida* (*Candida* spp.), for example, the non-glycosylated *C. albicans* Kre9 protein. As it is used in the present invention, the term "*C. albicans*" includes any strain of *Candida albicans,* a saprophytic diploid asexual fungus (form of yeast) of the family Saccharomycetaceae.

In a specific embodiment, the non-glycosylated protein of the invention is the non-glycosylated *C. albicans* Kre9 protein having the amino acid sequence shown in SEQ ID NO: 1 [Accession: XM_717838; Version: XM_717838.1, GI: 68465725; organism *C. albicans* SC5314]. In another specific embodiment, the non-glycosylated protein of the invention is the non-glycosylated *C. albicans* Kre9 protein having the amino acid sequence described in US 6,582,911 B1 (specifically in SEQ ID NO: 2 included in said United States patent).

The KRE9 gene of *C. albicans* encodes a protein (KRE9) similar to the product of the KRE9 gene of *Saccharomyces cerevisiae.* Its sequence has an open reading frame (ORF) of 813 base pairs (bp) which encodes a 29 kDa protein formed by 271 amino acids. The hydrophobic N-terminal region is a signal sequence approximately up to the point of binding between the amino acids 21 and 22. The *C. albicans* Kre9 protein contains a central area rich in serine and threonine residues characteristic of an O-glycosylation potential, typical of many wall mannoproteins. The disruption of the KRE9 gene of *C. albicans* shows that said Kre9 protein is required for the synthesis or assembly of β-1,6-glucans (Lussier et al., Proc Natl Acad Sci. 1998; 95:9825-9830). The homozygous mutant cells of *C. albicans* for the KRE9 gene grow poorly in galactose and are not capable of growing in filamentary form in serum and in media containing glucose the gene is essential. Example 2 illustrates the ability of a recombinant *C. albicans* Kre9 protein (CaKre9) expressed in *Escherichia coli* and, therefore, non-glycosylated, to significantly increase the adhesion of tumor cells (MB16 melanoma cells) to the hepatic sinusoidal endothelium (HSE).

In another particular embodiment, the non-glycosylated protein of the invention comprises or consists of a non-glycosylated variant of said protein having the amino acid sequence shown in SEQ ID NO: 1, and has at least the ability to increase the adhesion of tumor cells to the endothelium. As it is used in this description, the term "variant" relates to a peptide (or protein) substantially homologous and functionally equivalent to said protein comprising the amino acid sequence identified as SEQ ID NO: 1.

As it is used herein, a peptide is "substantially homologous" to said protein when its amino acid sequence has at least one 60% degree of identity, advantageously at least 70%, preferably at least 85%, and more preferably at least 95%, with respect to the amino acid sequence of said protein. Sequences homologous to a sequence of a protein can be easily identified by a person skilled in the art with the aid of a computer program suitable for comparing sequences, for example the BLAST program (Altschul et al. 1997. Nucleic Acids Res. 25:3389).

Likewise, as it is used herein, the expression "functionally equivalent" means that the peptide in question maintains the ability to increase the adhesion of tumor cells to the endothelium. The ability to increase the adhesion of tumor cells to the endothelium can be determined by means of any suitable assay known by the persons skilled in the art; for example, contacting tumor cells with endothelial cells stimulated with said non-glycosylated protein of the invention; to that end, said non-glycosylated protein of the invention binds to the suitable receptor and activates it, stimulating tumor adhesion to the endothelium. In a particular embodiment, the ability to increase the adhesion of tumor cells to the endothelium can be determined by means of an assay as described in Example 2.

In a specific embodiment, said variant is a mutant form of the protein comprising the amino acid sequence shown in SEQ ID NO: 1 which maintains the ability to increase the adhesion of tumor cells to the endothelium. Said mutant form may have insertions, deletions or modifications of one or more amino acid with respect to the protein comprised in SEQ ID NO: 1, with the proviso that the ability to increase the adhesion of tumor cells to the endothelium is conserved.

Likewise, as it is used in this description, the term "fragment" relates to a peptide comprising a portion of said protein comprising the amino acid sequence SEQ ID NO: 1, i.e., a contiguous amino acid sequence comprised within said SEQ ID NO: 1; for use thereof in the present invention said fragment must be functionally equivalent, i.e., the fragment (peptide) in question maintains the ability to increase the adhesion of tumor cells to the endothelium.

As previously mentioned, the non-glycosylated protein of the invention at least has the ability to increase the adhesion of tumor cells to the endothelium, an ability which can be determined by contacting the tumor cells with endothelial cells stimulated with said non-glycosylated protein of the invention by means of the binding or interaction of said non-glycosylated protein of the invention with the suitable receptor, the activation of which seems to stimulate tumor adhesion to the endothelium. Furthermore, the non-glycosylated protein of the invention may have the ability to bind to a suitable receptor, such as a cytokine receptor (thus mimicking the corresponding cytokine) or a receptor to which or with which the *C. albicans* Kre9 protein binds or interacts; some of said receptors can stimulate cell adhesion to the endothelium. In a particular embodiment, the receptor to which the non-glycosylated protein of the invention binds is a cytokine receptor, such as the interleukin-1 beta (IL-1β) receptor, so said non-glycosylated protein of the invention can act by mimicking said cytokine (IL-1β). The non-glycosylated protein of the invention can be used therefore in the identification of compounds potentially useful in the prevention and/or treatment of diseases associated with unwanted cell adhesion. The non-glycosylated protein of the invention can also have the ability to induce an inflammatory response, and therefore, due to its proinflammatory potential, acts as an antigen so it could be used in the prevention and/or treatment of a disease caused by an infectious agent.

The non-glycosylated protein of the invention can be obtained from an organism producing the glycosylated protein and by subjecting said glycosylated protein to a treatment to remove the carbohydrates, for example, by means of chemical oxidation (e.g., treatment with metaperiodate, etc.). Alternatively, the non-glycosylated protein of the invention can be obtained from an organism producing said non-glycosylated protein by means of a process which comprises cultivating said organism under suitable conditions for the expression of said protein and recovering it. In a particular embodiment of this invention, the organism producing the non-glycosylated protein of the invention is a prokaryotic organism comprising a polynucleotide which encodes the non-glycosylated protein of the invention; in a particular embodiment, said organism is a bacterium, e.g., *Escherichia coli*. Example 1 describes the production, isolation and purification of a non-glycosylated protein of the invention, specifically the recombinant *C. albicans* Kre9 protein (recombinant CaKre9 protein) comprising the amino acid sequence shown in SEQ ID NO: 1 (or a variant thereof) fused to an HSV tag and 6 histidine residues [(His)$_6$] with the ability to increase the adhesion of tumor cells to the endothelium.

Alternatively, the non-glycosylated protein of the invention can therefore be part of a fusion protein. In this sense, said fusion protein can contain, by way of non-limiting illustration, a region A formed by a first peptide comprising the non-glycosylated protein of the invention bound to a region B comprising a second peptide. Said second peptide can be any suitable peptide, for example, a peptide useful for identifying the recombinant protein, for example, by means of immunodetection of the recombinant protein (e.g., an HSV tag, or any other similar tag susceptible to being recognized, for example, by an antibody, etc.) and/or for purifying the recombinant protein, for example, a histidine tag which allows the purification of the recombinant protein by means of using an affinity column. Said region B can be bound to the amino-terminal region of said region A, or alternatively, said region B can be bound to the carboxyl-terminal region of said region A. Both regions A and B can be bound directly or through a linker peptide between said regions A and B. The fusion protein can be obtained by conventional methods known by the persons skilled in the art, for example by means of the genetic expression of the nucleotide sequence which encodes said fusion protein in suitable host cells.

The non-glycosylated protein of the invention can be found, if desired, in a composition comprising said non-glycosylated protein of the invention and an inert carrier. Said composition is an additional aspect of this invention. Virtually any inert carrier, i.e., a carrier that is not harmful for the non-glycosylated protein of the invention, can be used in the manufacture of said composition.

The nucleic acid sequence encoding said protein can be identified and isolated from the information provided by the non-glycosylated protein of the invention by means of using conventional techniques known by the persons skilled in the art, for example, by means of creating genomic DNA (DNAg) or complementary DNA (cDNA) libraries of organisms producing said protein; designing oligonucleotides suitable for amplification by means of polymerase chain reaction (PCR), a region of the genomic clone of the organisms producing said protein which serves for obtaining probes intended for scrutinizing said genomic libraries; and analyzing and selecting the positive clones. In a particular embodiment, a pair of oligonucleotide primers, consisting of the oligonucleotides the sequences of which are shown in SEQ ID NO. 3 and SEQ ID NO: 4, has been designed to amplify the gene encoding the *C. albicans* Kre9 protein. Said oligonucleotide primers are an additional aspect of this invention.

In a particular embodiment, the polynucleotide encoding the non-glycosylated protein of the invention comprises or consists of the nucleotide sequence shown in SEQ ID NO: 2. In a specific embodiment, the polynucleotide encoding the non-glycosylated *C. albicans* Kre9 protein having the amino acid sequence shown in SEQ ID NO: 2 [Accession: XM_717838; Version: XM_717838.1, GI: 68465725; organism *C. albicans* SC5314]. In another specific embodiment, the polynucleotide encoding the non-glycosylated *C. albicans* Kre9 protein has the nucleotide sequence described in US 6,582,911 B1 (specifically in SEQ ID NO: 1 included in said United States patent).

Alternatively, the polynucleotide encoding the non-glycosylated protein of the invention may have variations in its sequence with respect to the nucleotide sequence shown in SEQ ID NO: 2, for example, substitutions, insertions and/or deletions of one or more nucleotides, on the proviso that the resulting polynucleotide encodes a non-glycosylated protein of the invention. Therefore, polynucleotides substantially homologous to the polynucleotide, the nucleotide sequence of which is shown in SEQ ID NO: 2 and encodes a non-glycosylated protein of the invention, are within the scope of the present invention.

As it is used in this description, a polynucleotide is "substantially homologous" to the polynucleotide of SEQ ID NO: 2 when its nucleotide sequence has at least a degree of identity of at least 60%, advantageously at least 70%, preferably at least 85%, and more preferably at least 95%, with respect to the nucleotide sequence SEQ ID NO: 2. A polynucleotide substantially homologous to the polynucleotide of SEQ ID NO: 2 can typically be isolated from a organism producing the glycosylated/non-glycosylated protein of the invention based on the information contained in said SEQ ID NO: 2, or can also be formed based on the DNA sequence shown in SEQ ID NO: 2, for example, by means of introducing conservative or non-conservative substitutions. Other examples of possible modifications include the insertion of one or more nucleotides in the sequence, the addition of one or more nucleotides in any of the ends of the sequence, or the deletion of one or more nucleotides in any end or within the sequence.

Said polynucleotide encoding the non-glycosylated protein of the invention can be contained in a gene construct comprising said polynucleotide. Said gene construct can incorporate, operatively bound thereto, a regulatory sequence for the expression of said polynucleotide encoding the non-glycosylated protein of the invention, thus forming an expression cassette. As it is used in this description, the expression "operatively bound" means that the non-glycosylated protein of the invention, encoded by said polynucleotide, is expressed in the correct reading frame under the control of the control sequences or the expression regulators. The control sequences are sequences that control and regulate the transcription and, where appropriate, the translation of the protein of the invention and include promoter sequences, transcription regulator encoding sequences, ribosome binding sequences (RBS) and/or transcription termination sequences. In a particular embodiment, said expression control sequence is functional in prokaryotic organisms, for example, bacteria, etc., such as *E. coli* or the like. Advantageously, the construct further comprises a marker or gene encoding a motif or a phenotype which allows the selection of the organism transformed with said construct. Said gene construct can be obtained by means of using widely known techniques in the state of the art (Sambrook et al., "Molecular cloning, a Laboratory Manual", 2nd ed., Cold Spring Harbor Laboratory Press, N.Y., 1989 Vol 1-3).

Likewise, said gene construct can be inserted in a suitable vector. The choice of the vector will depend on the host organism in which said vector is going to be subsequently introduced. In a particular embodiment, the vector is a plasmid (pETKRE9) from a commercial pET-Blue plasmid (Example 1). Said vector can be obtained by conventional methods known by the persons skilled in the art (Sambrook *et al*., mentioned above). In a particular embodiment, said vector is a vector useful for transforming bacteria, e.g., competent *E. coli.*

Said vector can be used for transforming organisms susceptible of being transformed by said vector. Given that the protein to be expressed (non-glycosylated protein of the invention) should not be glycosylated, said organisms are prokaryotic organisms, for example, bacteria, such as *E. coli,* etc., e.g., *E. coli* BL21(DE3) (Studier and Moffatt (1986) J. Mol. Biol. 189: 113-130), etc.

Therefore, in another aspect, the invention relates to a prokaryotic organism, hereinafter "prokaryotic organism of the invention", transformed with the aforementioned vector; therefore, the prokaryotic organism of the invention comprises, therefore, a polynucleotide encoding a non-glycosylated protein of the invention, or a gene construct comprising said polynucleotide, or an expression cassette comprising said polynucleotide, or a vector comprising said polynucleotide, and is capable of expressing the non-glycosylated protein of the invention. As previously mentioned, said prokaryotic organism of the invention is a bacterium, for example, *E. coli,* etc., such as *E. coli* BL21(DE3), etc. The prokaryotic organism of the invention can be obtained by conventional methods known by persons skilled in the art [Sambrok et al., 1989, mentioned above].

In another aspect, the invention relates to a process for obtaining a non-glycosylated protein of the invention, which comprises cultivating a prokaryotic organism of the invention under conditions that allow the production of said protein and, if desired, recovering said non-glycosylated protein of the invention from the culture medium. The conditions for optimizing the cultivation of said prokaryotic organism of the invention will depend on the prokaryotic organism used; said conditions are known by the persons skilled in the art. The process for producing the non-glycosylated protein of the invention optionally includes isolating and purifying said non-glycosylated protein of the invention.

As previously mentioned, the non-glycosylated protein of the invention has the ability to increase the adhesion of tumor cells to the endothelium and the ability to bind to a suitable receptor, such as a cytokine receptor (thus mimicking the corresponding cytokine) or a receptor to which or with which the *C. albicans* Kre9 protein binds or interacts, for example, receptors that can stimulate cell adhesion to the endothelium, such as the IL-1β receptor, so said non-glycosylated protein of the invention can act by mimicking said cytokine (IL-1β). Therefore, the non-glycosylated protein of the invention can be used in the identification of compounds potentially useful in the prevention and/or treatment of a disease associated with unwanted cell adhesion.

In the context of the present invention, a "disease associated with unwanted cell adhesion" includes the progression and metastasis of cancer and tumors. Non-limiting illustrative examples of diseases associated with unwanted cell adhesion which can be treated according to the teachings of the present invention include cancer, metastasis, restenosis, diseases which stimulate a cellular immune response and inflammatory diseases.

In a particular embodiment of the invention, the disease associated with unwanted cell adhesion is cancer or a tumor or cancerous process, i.e., a physiological condition in mammals characterized by deregulated cell growth.

In another particular embodiment of the invention, the disease associated with unwanted cell adhesion is a metastasis or metastatic process, i.e., the propagation of a cancerous focus to an organ which is different from that in which it started, for example, through the blood or lymphatic system. In a particular embodiment, said metastatic process leads to the implantation of a tumor in the liver, for example, in the HSE.

In another particular embodiment of the invention, the disease associated with unwanted cell adhesion is restenosis, i.e., a disease wherein a excessive migration of cells occurs resulting from the release of growth factors produced by mechanical damage of the endothelial cells forming the coronary arteries.

In another particular embodiment of the invention, the disease associated with unwanted cell adhesion is a disease which stimulates a cellular immune response, i.e., a form of adaptive immune response mediated by T-lymphocytes which act as a defense mechanism against intracellular microorganisms, e.g., viruses, some bacteria and fungi, capable of surviving and proliferating in phagocytes and other cells of the host, a place to which the circulating antibodies have no access, etc. The defense against infections of this type depends on the cellular immunity which induces the destruction of the microorganism residing in the phagocytes or of the infected cells.

In another particular embodiment of the invention, the disease associated with unwanted cell adhesion is an inflammatory disease or inflammatory process, particularly an inflammatory process mediated by cytokines, i.e., a disease which stimulates inflammation as a consequence of cell proliferation associated with a proliferative dysfunction, e.g., proliferative glomerulonephritis, lupus erythematosus, scleroderma, temporal arthritis, thromboangiitis, mucocutaneous lymph node syndrome, etc.

Identification of potentially therapeutic compounds

Therefore, in another aspect, the invention relates to an in vitro method for identifying a compound potentially useful for the prevention and/or treatment of a disease associated with unwanted cell adhesion, hereinafter method for identifying (I) potentially therapeutic compounds of the invention, which comprises:

a) contacting a cell which expresses a receptor to which or with which a non-glycosylated protein of the invention binds or interacts with a non-glycosylated protein of the invention and with the candidate compound, and
b) identifying those compounds which inhibit the binding of said non-glycosylated protein of the invention to said receptor.

In a particular embodiment, the method for identifying potentially therapeutic compounds of the invention is particularly useful and is suitable for identifying and selecting compounds potentially useful for the prevention and/or treatment of tumor processes, metastatic processes, restenosis, diseases which provoke a cellular immune response and/or inflammatory diseases.

Although virtually any cell which expresses a receptor to which or with which a non-glycosylated protein of the invention binds or interacts can be used in the implementation of said method, in a particular embodiment, said cell which expresses a receptor to which or with which a non-glycosylated protein of the invention binds or interacts is a cell expressing a cytokine receptor, e.g., an IL-1β receptor, or a receptor of any other cytokine, or generally a cell expressing a receptor to which the *C. albicans* Kre9 protein binds; thus, in a particular embodiment, said cell which expresses a receptor to which or with which a non-glycosylated protein of the invention binds or interacts is a suitable cell from the endothelium, for example, a cell from the hepatic sinusoidal endothelium (HSE).

Treatment and/or prevention of diseases associated with unwanted cell adhesion

The compounds identified according to the method for identifying potentially therapeutic compounds of the invention can be used in the prevention and/or treatment of a disease associated with unwanted cell adhesion. Therefore, in another aspect, the invention relates to an inhibitory agent of the activity of the non-glycosylated protein of the invention for the prevention and/or treatment of a disease associated with unwanted cell adhesion, such as a tumor process, a metastatic process, restenosis, a disease which provokes a cellular immune response and/or an inflammatory disease. Therefore, in other words, in another aspect, the invention relates to the use of an inhibitory agent of the activity of the non-glycosylated protein of the invention in the manufacture of a pharmaceutical composition for the prevention and/or treatment of a disease associated with unwanted cell adhesion, such as a tumor process, a metastatic process, restenosis, a disease which provokes a cellular immune response and/or an inflammatory disease.

In another aspect, the invention relates to a method for the prevention and/or treatment of a disease associated with unwanted cell adhesion, such as a tumor process, a metastatic process, restenosis, a disease which stimulates a cellular immune response and/or an inflammatory disease, which comprises administrating a therapeutically effective amount of an inhibitory agent of the activity of a non-glycosylated protein of the invention (occasionally referred to as "inhibitory agent (I) of the invention") to a subject in need of treatment.

As it is used herein, the term "subject" relates to a member of a mammal species, including but not limited to domestic animals, primates and humans; preferably, the subject is a human being, male or female, of any age or race. In a particular embodiment, said subject in need of treatment is a patient with cancer; in another specific embodiment, said subject in need of treatment is a patient with cancer who further suffers a disease caused by an infectious agent, for example, candidiasis.

In the context of the present invention, the term "non-glycosylated protein of the invention" has already been previously defined. In a particular embodiment of the invention, the non-glycosylated protein of the invention comprises or consists of the amino acid sequence shown in SEQ ID NO: 1, or the non-glycosylated Candida spp. Kre9 protein, e.g., the non-glycosylated *C. albicans* Kre9 protein, such as the non-glycosylated *C. albicans* Kre9 protein having the amino acid sequence shown in SEQ ID NO: 2 of United States patent US 6,582,911 B1. In another particular embodiment of the invention, the non-glycosylated protein of the invention is a functionally equivalent variant or fragment of the aforementioned non-glycosylated proteins.

In the context of the present invention, "inhibitory agent (I) of the invention" is understood as any substance or compound capable of preventing or blocking the non-glycosylated protein of the invention from performing its function (activity), i.e., preventing said non-glycosylated protein of the invention from activating/stimulating the adhesion of tumor cells to the endothelium. Although it is not intended to be bound by any theory, it is believed that said activity for stimulating tumor adhesion to the endothelium can be performed by means of the non-glycosylated protein of the invention activating the suitable receptors (e.g., the IL-1β receptor, etc.), so the binding of a compound to said non-glycosylated protein of the invention can result in the non-glycosylated protein of the invention not being able to bind to the receptor or receptors thereof. Assays to determine whether a compound is an inhibitory agent of a non-glycosylated protein of the invention can be designed by persons skilled in the art from the information provided by this invention, for example, from the information contained in Example 2.

By way of non-limiting illustration, inhibitory agents of the activity of the non-glycosylated protein of the invention for use thereof in the present invention include, for example, inhibitory peptides of said non-glycosylated protein of the invention, antibodies directed specifically against epitopes of said non-glycosylated protein of the invention which are essential for carrying out the function thereof, etc. Therefore, in a particular embodiment of the invention, said inhibitory agent (I) of the invention is selected from the group consisting of inhibitory peptides and antibodies.

As it is used herein, the term "inhibitory peptide" relates to those peptides capable of binding to a non-glycosylated protein of the invention and inhibiting its activity such as hereinbefore explained, i.e., preventing the non-glycosylated protein of the invention from being able to induce or stimulate the adhesion of tumor cells to the endothelium.

In the context of the present invention, "inhibitory antibody" is understood as any antibody that is capable of binding to a non-glycosylated protein of the invention, preventing said non-glycosylated protein of the invention from being able to induce or stimulate the adhesion of tumor cells to the endothelium. Said antibodies can be prepared using any of the methods that are known by the person skilled in the art. Thus, polyclonal antibodies are prepared by means of immunizing an animal with the non-glycosylated protein of the invention to be inhibited. Monoclonal antibodies are prepared by using the method described by Köhler and Milstein (Nature, 1975; 256:495-397) or by Harlow and Lane ("Using Antibodies. A Laboratory Manual" by E. Harlow and D. Lane, Editor: Cold Spring Harbor Laboratory Press, Cold Spring Harbor, New York; 1998 (ISBN 978-0879695439)). Once the antibodies with the ability to bind to a non-glycosylated protein of the invention are identified, those that are capable of inhibiting the activity of said protein will be selected using the inhibitory agent identification assay which has been referred to above.

In a particular embodiment, said inhibitory antibody is an antibody which specifically recognizes a non-glycosylated protein of the invention, such as a non-glycosylated protein comprising or consisting of the amino acid sequence shown in SEQ ID NO: 1, or the non-glycosylated Candida spp. Kre9 protein, e.g., the non-glycosylated *C. albicans* Kre9 protein, such as the non-glycosylated *C. albicans* Kre9 protein having the amino acid sequence shown in SEQ ID NO: 2 of United States patent US 6,582,911 B1. In another particular embodiment, said inhibitory antibody is an antibody which specifically recognizes a functionally equivalent variant or fragment of the aforementioned non-glycosylated proteins. In a particular embodiment, said inhibitory antibody is an antibody which recognizes IL-1β, e.g., an anti-mammal IL-1β antibody.

In the present invention, the term "antibody" must be interpreted broadly and includes polyclonal antibodies, monoclonal antibodies, multispecific antibodies and fragments thereof (F(ab')2, Fab), etc. provided that they are capable of specifically recognizing the antigen of interest, which in the context of the present invention is a non-glycosylated protein of the invention. Examples of antibodies that can be used in the context of the present invention are, as non-limiting examples, polyclonal antibodies, monoclonal antibodies, recombinant antibodies, chimeric antibodies, humanized antibodies, completely human antibodies, etc.

Polyclonal antibodies are originally heterogeneous mixtures of antibody molecules produced in the serum of animals that have been immunized with an antigen. They also include monospecific polyclonal antibodies obtained from the heterogeneous mixtures, for example, by means of column chromatography with peptides having a single epitope of the antigen of interest.

A monoclonal antibody is a homogenous population of antibodies specific for a single epitope of the antigen. These monoclonal antibodies can be prepared by means of already described conventional techniques (see above).

A chimeric antibody is an antibody monoclonal constructed by means of cloning or recombining antibodies from different animal species. In a non-limiting typical configuration of the invention, the chimeric antibody includes part of a monoclonal antibody, generally the variable region (Fv) which includes the antigen recognition and binding sites, and the other part corresponding to a human antibody, generally the part which includes the constant region and the adjacent constant region.

A completely human antibody is an antibody or antibodies that have been produced in transgenic animals with a human immune system or by *in vitro* immunization of human immune cells (including both genetic immunization and traditional immunization with or without adjuvants and pure or non-pure antigen; or by means of any method for exposing the antigen to the immune system) or by means of native/synthetic libraries produced from human immune cells. These antibodies can be obtained and selected from transgenic animals (for example mice) into which the genes of human immunoglobulins have been cloned and which are immunized with the target antigen (in the present invention said antigen is a non-glycosylated protein of the invention). These antibodies can be obtained by selecting the human single-chain variable fragment (scFv) or the fragment antigen-binding (Fab) presented in phage displays libraries and subsequent cloning and insertion in a human antibody or by means of any other method for producing and displaying, known by the person skilled in the art, the libraries generated by cloning the variable regions of both chains and subsequent combination/mutation of the latter to generate antibody libraries.

A humanized antibody is a monoclonal antibody constructed by means of cloning and grafting the hypervariable complementarity determining regions (CDR) of a murine monoclonal antibody into a human antibody substituting its own hypervariable CDR regions.

In addition, in the context of the present invention, variants with an altered glycosylation pattern are also included within the term "antibody", as are glycosylated or non-glycosylated antibody fragments obtained from the protein or by means of recombinant technology, which may consist (i) of variable areas of the antibodies bound to one another by a binding peptide (scFv), (ii) of the variable area next to the CH1 constant of the heavy chain (Fd) bound to the light chain by means of cysteines or by means of binding peptides and disulfide bond (scFab), (iii) new variants, such as the heavy chains alone, or (iv) any modification that is made on the antibody fragments for the purpose of making them more similar, less immunogenic (humanized) or more stable in biological fluids and which, in the context of the present invention, have the ability to prevent the non-glycosylated proteins of the invention from performing their function (activity), i.e., inducing or stimulating the adhesion of tumor cells to the endothelium.

The person skilled in the art will understand that the antibodies can be obtained by means of conventional genetic engineering or recombinant techniques, techniques for producing, extracting and purifying antibodies from biological tissues or fluids, or by any other conventional technique for obtaining proteins and antibodies which are widely know by the person skilled in the art. Non-limiting illustrative examples of techniques for producing antibodies are: animal immunization techniques including transgenic animals for human immunoglobulin genes, producing monoclonal antibodies by means of hybridomas, producing by means of antibody libraries which may be native, synthetic or derived from organisms immunized against the antigen of interest and which may be selected by means of very different methods of display (phage display, ribosome display, etc.), and subsequently by means of genetic engineering techniques, antibodies may be redesigned and expressed in vectors designed for producing recombinant antibodies of different sizes, composition and structure. A review of the main methods for producing and purifying purification of antibodies can be found, for example, in:

- "Handbook of Therapeutic Antibodies", S. Dübel. Editor: Wiley-VCH, 2007, Vol: I to III (ISBN 978-3527314539);
- "Antibodies: Volume 1: Production and Purification", G. Subramanian Ed., Editor: Springer, 1st Ed, 2004 (ISBN 978-0306482458);
- "Antibodies: Volume 2: Novel Technologies and Therapeutic Use", G. Subramanian Ed., Editor: Springer, first edition, 2004 (ISBN 978-0306483158); or
- "Molecular Cloning: a Laboratory Manual", J. Sambrook and D.W. Russel Eds., Publisher: Cold Spring Harbor Laboratory Press, third edition, 2001 (ISBN 978-0879695774).

As described in the beginning of the description, the inventors of this invention have opened up a new therapeutic window in the treatment of diseases associated with unwanted cell adhesion, such as tumor processes (cancer), metastatic processes (metastasis), restenosis, diseases which stimulate a cellular immune response and/or inflammatory diseases.

Therefore, in another aspect, the invention relates to a pharmaceutical composition, hereinafter "pharmaceutical composition A of the invention", comprising a therapeutically effective amount of an inhibitory agent (I) of the invention together with a pharmaceutically acceptable carrier for the treatment of diseases associated with unwanted cell adhesion. Examples of diseases associated with unwanted cell adhesion include tumor processes (cancer), metastatic processes (metastasis), restenosis, diseases which stimulate a cellular immune response and/or inflammatory diseases and they have been mentioned above in the present description.

"Therapeutically effective amount" is understood in the context of the present invention as the amount of inhibitory agent (I) of the invention necessary to achieve the desired effect which in this specific case, is the treatment of diseases associated with unwanted cell adhesion. Generally, the therapeutically effective amount of the inhibitory agent (I) of the invention to be administered will depend, among other factors, on the subject that will be treated, the severity of the disease that said subject suffers, the chosen method of administration, etc. For this reason, the doses mentioned in this invention must be considered only as guidelines for the person skilled in the art, and the latter must adjust the dose according to the aforementioned variables. Nevertheless, an inhibitory agent (I) of the invention can be administered once or more times a day, for example, 1, 2, 3 or 4 times a day, in a typical total daily amount comprised between 0.1 and 1.000 mg/kg of body mass/day, preferably 10 mg/kg of body mass/day.

The term "treatment" or "treating" in the context of this description means the administration of an inhibitory agent (I) of the invention for preventing, relieving or curing the disease, or one or more symptoms associated with said disease associated with unwanted cell adhesion. "Treatment" also covers preventing, relieving or eliminating the physiological sequelae of the disease. The term "relieving" in the context of this invention is understood as any improvement of the situation of the treated patient - both subjectively (the feeling of or about the patient) and objectively (measured parameters).

The term "vehicle, adjuvant and/or carrier" relates to molecular entities or substances with which the active ingredient (inhibitory agent (I) of the invention) is administered. Such pharmaceutical vehicles, adjuvants or carriers can be sterile liquids, such as water and oils including those of petroleum or of animal, plant or synthetic origin, such as peanut oil, soybean oil, mineral oil, sesame oil and the like, excipients, disintegrating agents, wetting agents or diluting agents. Suitable pharmaceutical transporters are described in "Remington's Pharmaceutical Sciences", E.W. Martin.

In the context of the present invention, the term "pharmaceutically acceptable" relates to molecular entities and compositions that are physiologically tolerable and do not typically produce an allergic reaction or a similar unfavorable reaction such as stomach disorder, dizziness and the like, when administered to a human. Preferably, the term "pharmaceutically acceptable" means it is approved by a regulatory agency of a state or federal government or included in the United States Pharmacopoeia or other generally recognized pharmacopoeia for use in animals, and more particularly in humans.

The inhibitory agent (I) of the invention as well as the pharmaceutical compositions containing it can be used together with other additional drugs useful in the treatment of diseases associated with unwanted cell proliferation and/or adhesion. Said additional drugs can be part of the same pharmaceutical composition A of the invention, or they can alternatively be provided in the form of a separate pharmaceutical composition for the simultaneous or non-simultaneous administration thereof with respect to that of pharmaceutical composition A of the invention comprising said inhibitory agent (I) of the activity of a non-glycosylated protein of the invention.

Examples of other additional drugs useful in the treatment of diseases associated with unwanted cell proliferation are but are not limited to alkylating agents such as, for example, cyclophosphamide, carmustine, daunorubicin, mechlorethamine, chlorambucil, nimustine, melphalan and the like; anthracyclines, such as, for example, daunorubicin, doxorubicin, epirubicin, idarubicin, mitoxantrone, valrubicin and the like; taxane compounds, such as, for example, paclitaxel, docetaxel and the like; topoisomerase inhibitors such as, for example, etoposide, teniposide, tuliposide, irinotecan and the like; nucleotide analogues such as, for example, azacitidine, azathioprine, capecitabine, citarabine, doxifluridine, fluorouracil, gemcitabine, mercaptopurine, methotrexate, thioguanine, ftorafur and the like; platinum-based agents such as, for example, carboplatin, cisplatin, oxaliplatin and the like; anti-neoplastic agents such as, for example, vincristine, leucovorin, lomustine, procarbazine and the like; hormone modulator such as, for example, tamoxifen, finasteride, 5-$\alpha$-reductase inhibitors and the like; vinca alkaloids such as, for example, vinblastine, vincristine, vindesine, vinorelbine and the like. The suitable chemotherapeutic agents are described in further detail in the literature, such as in The Merck Index in CD-ROM, 13th edition.

Induction of an inflammatory response Alternatively, the inhibitory agent (I) of the invention as well as the pharmaceutical compositions containing it can be used together with other additional drugs useful in the treatment of fungal infections, for example, antifungal agents useful in the prevention and/or treatment of candidiasis. Said additional drugs can be part of the same pharmaceutical composition A of the invention, or they can alternatively be provided in the form of a separate composition for the simultaneous or non-simultaneous administration thereof with respect to that of pharmaceutical composition A of the invention comprising said inhibitory agent (I) of the activity of a non-glycosylated protein of the invention.

Examples of drugs useful in the treatment of fungal infections include but are not limited to (i) polyenes, such as nystatin, natamycin, amphotericin B, etc., (ii) azoles, such as imidazoles (miconazole, clotrimazole), triazoles (fluconazole, itraconazole, ketoconazole), second-generation triazoles (voriconazole, ravuconazole, posaconazole), etc., (iii) allylamines, such as terbinafine, naftifine, etc., (iv) lipopeptides, such as papulacandins, glycosylated triterpenes, echinocandins (caspofungin, anidulafungin, micafungin), etc., and (v) fluorinated pyrimidines, such as flucytosine, etc. Other antifungal agents include, for example, potassium iodide, ciclopirox, tolnaftate, griseofulvin, etc.

Pharmaceutical composition A of the invention can be administered by any suitable administration route, for example, orally, parenterally (for example, subcutaneously, intraperitoneally, intravenously, intramuscularly, etc.), rectally, etc., it is typically administered orally due to the chronic character of the disease to be treated.

Illustrative examples of pharmaceutical dosage forms by oral route include tablets, capsules, granules, solutions, suspensions, etc., and they can contain the suitable excipients, such as binding agents, diluting agents, disintegrating agents, lubricants, wetting agents, etc., and can be prepared by conventional methods. The pharmaceutical compositions can also be adapted for parenteral administration in the form of, for example, sterile solutions, suspensions or lyophilized products, in the suitable dosage form; in this case, said pharmaceutical compositions will include the suitable excipients, such as buffers, surfactants, etc. In any case, the excipients will be chosen according to the selected pharmaceutical dosage form.

A review of the different pharmaceutical dosage forms of drugs and the manufacture thereof can be found in the book "Tratado de Farmacia Galénica", by C. Faulí i Trillo, 10th Edition, 1993, Luzán 5, S.A. de Ediciones.

Likewise, as has been mentioned above, the non-glycosylated protein of the invention may have the ability to induce an inflammatory response, so, due to its proinflammatory potential, said non-glycosylated protein of the invention can act as an antigen, and could be used as such in the prevention and/or treatment of a disease caused by an infectious agent. Although it is not intended to be bound by any theory, it is believed that the recombinant *C. albicans* Kre9 protein (Example 2), a representative example of a non-glycosylated protein of the invention, presumably acts by binding to the IL-1β receptor, so it has a higher proinflammatory potential due to its ability to bind to and activate said receptor, acting as if it was endogenous IL-1β, one of the most important proinflammatory cytokines in the immune response.

Therefore, in another aspect, the invention relates to a pharmaceutical composition, hereinafter "pharmaceutical composition B of the invention", comprising a therapeutically effective amount of a non-glycosylated protein of the invention together with a pharmaceutically acceptable carrier for the prevention and/or treatment of a disease caused by an infectious agent.

As it is used in this description, a "disease caused by an infectious agent" relates to the clinical manifestation resulting from an infection stimulated by microorganisms, e.g., bacteria, fungi, viruses, protozoa, etc., or by prions. In a particular embodiment, said infectious agent is an infectious agent which expresses the *C. albicans* Kre9 protein or a protein similar to said *C. albicans* Kre9 protein which may influence the inflammatory response, for example, a species of the genus Candida, a related fungus which may be spread through blood, etc. Likewise, in another particular embodiment, said infectious agent is a microorganism which can be spread through the blood stream and adhere itself to organs activating an inflammatory and immune response. In a specific embodiment, said disease caused by an infectious agent is candidiasis or a group of infections caused by an opportunistic fungus of the genus *Candida, C. albicans* being the most frequent, which infections can be expressed cutaneously, gastrointestinally, in the respiratory system and in the genitals. These fungi are always present in the skin and in the mucus of the digestive, urinary and respiratory tracts of most people, they are controlled by other non-pathogenic microorganisms. When an imbalance occurs, the excessive increase of the fungus population produces this or other mycoses.

The terms "treating" or "treatment", "therapeutically effective amount", "non-glycosylated protein of the invention" and "pharmaceutically acceptable carrier" have already been previously defined in this description and are applicable to pharmaceutical composition B of the invention, as well as everything related to the dosage form of pharmaceutical composition A of the invention. In this sense, pharmaceutical composition B of the invention can be administered by any suitable administration route, for example, orally, parenterally (for example, subcutaneously, intraperitoneally, intravenously, intramuscularly, etc.), rectally, etc. Illustrative examples of pharmaceutical dosage forms by oral route include tablets, capsules, granules, solutions, suspensions, etc., they contain the suitable excipients, such as binding agents, diluting agents, disintegrating agents, lubricants, wetting agents, etc., and can be prepared by conventional methods. The pharmaceutical compositions can also be adapted for parenteral administration in the form of, for example, sterile solutions, suspensions or lyophilized products, in the suitable dosage form; in this case, said pharmaceutical compositions will include the suitable excipients, such as buffers, surfactants, etc. In any case, the excipients will be chosen according to the selected pharmaceutical dosage form. A review of the different pharmaceutical dosage forms of drugs and the manufacture thereof can be found in the book "Tratado de Farmacia Galénica", by C. Faulí i Trillo, 10th Edition, 1993, Luzán 5, S.A. de Ediciones.

In a particular embodiment, said non-glycosylated protein of the invention present in pharmaceutical composition B of the invention is a non-glycosylated protein comprising or consisting of the amino acid sequence shown in SEQ ID NO: 1, or the non-glycosylated Candida spp. Kre9 protein, e.g., the non-glycosylated *C. albicans* Kre9 protein, such as the non-glycosylated *C. albicans* Kre9 protein having the amino acid sequence shown in SEQ ID NO: 2 of United States patent US 6,582,911 B1. In another particular embodiment, said non-glycosylated protein of the invention is a functionally equivalent variant or fragment of the aforementioned non-glycosylated proteins.

The non-glycosylated protein of the invention as well as the pharmaceutical compositions containing it can be used together with other additional drugs useful in the treatment of diseases caused by one or more infectious agents. Said additional drugs can be part of pharmaceutical composition B of the invention, or they can alternatively be provided in the form of a separate pharmaceutical composition for the simultaneous or non-simultaneous administration thereof with respect to that of pharmaceutical composition B of the invention comprising said non-glycosylated protein of the invention.

Examples of other additional drugs useful in the treatment of diseases caused by infectious agents include antimicrobial agents, antibacterial agents, antiviral agents, antiparasitic agents, antifungal agents, antibiotics, etc. By way of non-limiting illustration, examples of additional drugs useful in the treatment of fungal infections include but are not limited to (i) polyenes, such as nystatin, natamycin, amphotericin B, etc., (ii) azoles, such as imidazoles (miconazole, clotrimazole), triazoles (fluconazole, itraconazole, ketoconazole), second-generation triazoles (voriconazole, ravuconazole, posaconazole), etc., (iii) allylamines, such as terbinafine, naftifine, etc., (iv) lipopeptides, such as papulacandins, glycosylated triterpenes, echinocandins (caspofungin, anidulafungin, micafungin), etc., and (v) fluorinated pyrimidines, such as flucytosine, etc. Other antifungal agents include, for example, potassium iodide, ciclopirox, tolnaftate, griseofulvin, etc.

In another aspect, the invention relates to a non-glycosylated protein of the invention for the prevention and/or treatment of a disease caused by an infectious agent; i.e., in another aspect, the invention relates to the use of a non-glycosylated protein of the invention in the manufacture of a pharmaceutical composition for the prevention and/or treatment of a disease caused by an infectious agent. In a particular embodiment, said disease caused by an infectious agent is a disease caused by microorganisms, e.g., bacteria, fungi, viruses, protozoa, etc., or by prions. In a particular embodiment, said infectious agent is an infectious agent which expresses the *C. albicans* Kre9 protein or a protein similar to said *C. albicans* Kre9 protein that may influence the inflammatory response, for example, a species of the genus *Candida,* a related fungus which may be spread through the blood, etc. Likewise, in another particular embodiment, said infectious agent is a microorganism that can be spread through the blood stream and adhere to organs activating an inflammatory and immune response. In a specific embodiment, said disease caused by an infectious agent is candidiasis.

In a particular embodiment, said non-glycosylated protein of the invention present in pharmaceutical composition B of the invention is a non-glycosylated protein comprising or consisting of the amino acid sequence shown in SEQ ID NO: 1, or the non-glycosylated Candida spp. Kre9 protein, e.g., the non-glycosylated *C. albicans* Kre9 protein, such as the non-glycosylated *C. albicans* Kre9 protein having the amino acid sequence shown in SEQ ID NO: 2 of United States patent US 6,582,911 B1. In another particular embodiment, said non-glycosylated protein of the invention is a functionally equivalent variant or fragment of the aforementioned non-glycosylated proteins.

In another aspect, the invention relates to a method for the prevention and/or treatment of a disease caused by an infectious agent, which comprises administrating a therapeutically effective amount of a non-glycosylated protein of the invention to a subject in need of treatment.

In another aspect, the invention relates to the use of a kit comprising:

an inhibitory agent (I) of the invention (for example, a compound capable of binding to a non-glycosylated protein of the invention and blocking the binding of said protein to its receptor or
receptors present in endothelial cells) for the prevention and/or treatment of a disease associated with unwanted cell adhesion,
or alternatively,
a non-glycosylated protein of the invention for the prevention and/or treatment of a disease caused by an infectious agent.

In the present invention, a "kit" is understood as a product which contains the different products (e.g., inhibitory agent (I) of the invention or non-glycosylated protein of the invention) forming the composition packaged such that it allows transporting, storing and simultaneous or sequential administration thereof. The kits of the invention can thus contain one or more suspensions, tablets, capsules, inhalers, syringes, patches and the like, which contain said products and which can be prepared in an individual dose or as multiple doses. The kit can additionally contain a suitable support for resuspending the compositions of the invention such as an aqueous medium, such as saline solution, Ringer's solution, lactated Ringer's solution, dextrose and sodium chloride, water soluble media, such as alcohol, polyethylene glycol, propylene glycol and water insoluble supports such as corn oil, cottonseed oil, peanut oil, sesame oil, ethyl oleate, isopropyl myristate and benzyl benzoate. Other components which can be present in the kit is a container which allows maintaining the formulations of the invention within determined limits. The materials suitable for preparing such containers include glass, plastic, polyethylene, polypropylene, polycarbonate and the like, bottles, vials, paper, sachets and the like.

The kit of the invention can additionally contain instructions for the simultaneous, sequential or separate administration of the different pharmaceutical formulations present in the kit. Therefore, in a particular embodiment the kit of the invention further comprises instructions for the simultaneous, sequential or separate administration of the different components. Said instructions can be found as printed material or as electronic medium which can store the instructions such that they can be read by a subject, such as electronic storing means (magnetic discs, cassettes and the like), optical means (CD-ROM, DVD) and the like. The means can additionally or alternatively contain Internet providing said instructions.

All the particular embodiments defined by this inventive aspect have been mentioned, described and explained in previous inventive aspects, therefore it is not necessary to repeat them.

II. Applications of the glycosylated protein of the invention

The invention also relates to the applications of a glycosylated protein comprising the amino acid sequence shown in SEQ ID NO: 1, or a functionally equivalent variant or fragment thereof, capable of increasing the adhesion of tumor cells to the endothelium.

For the sake of simplicity, said glycosylated protein comprising the amino acid sequence identified as SEQ ID NO: 1, as well as the functionally equivalent variants and fragments thereof capable of increasing the adhesion of tumor cells to the endothelium is included under the designation "glycosylated protein of the invention".

In a particular embodiment, the glycosylated protein of the invention comprises or consists of the amino acid sequence shown in SEQ ID NO: 1 which contains a central area rich in serine and threonine residues characteristic of an O-glycosylation potential; said protein at least has the ability to increase the adhesion of tumor cells to the endothelium, which ability can be determined by contacting tumor cells with endothelial cells stimulated with said glycosylated protein of the invention.

As it is used in this description, "glycosylated protein of the invention" relates to said protein comprising or consisting of the amino acid sequence shown in SEQ ID NO: 1, containing at least one sugar residue in any of the potential glycosylation sites, for example, in any of the serine or threonine residues characteristic of an O-glycosylation potential present in said protein.

In a particular embodiment, the glycosylated protein of the invention is the glycosylated Kre9 protein of a species of the genus Candida (*Candida* spp.), for example, the non-glycosylated *C. albicans* Kre9 protein. In a specific embodiment, the glycosylated protein of the invention is the native *C. albicans* Kre9 protein (glycosylated) having the amino acid sequence shown in SEQ ID NO: 1 [Accession: XM_717838; Version: XM_717838.1, GI: 68465725; organism *C. albicans* SC5314]. In another specific embodiment, the glycosylated protein of the invention is the glycosylated *C. albicans* Kre9 protein having the amino acid sequence described in US 6,582,911 B1 (specifically in SEQ ID NO: 2 included in said United States patent).

Various assays performed by the inventors have shown that not only is the recombinant *C. albicans* Kre9 (CaKre9) protein expressed in *E.coli* and, therefore, non-glycosylated, capable of significantly increasing the adhesion of tumor cells (MB16 melanoma cells) to the hepatic sinusoidal endothelium (HSE), but the native *C. albicans* Kre9 protein is as well.

In another particular embodiment, the glycosylated protein of the invention comprises or consists of a glycosylated variant (i.e., it contains at least one sugar residue in any of the potential glycosylation sites) of said protein having the amino acid sequence shown in SEQ ID NO: 1, and which at least has the ability to increase the adhesion of tumor cells to the endothelium. As it is used in this description, the term "variant" relates to a peptide (or protein) substantially homologous and functionally equivalent to said protein comprising the amino acid sequence identified as SEQ ID NO: 1. The expressions "substantially homologous" and "functionally equivalent", when applied to proteins and peptides, have previously been. The ability to increase the adhesion of tumor cells to the endothelium can be determined by means of any suitable assay known by the persons skilled in the art; for example, contacting tumor cells with endothelial cells stimulated with said glycosylated protein of the invention; to that end, said glycosylated protein of the invention binds to the suitable receptor and activates it, stimulating the tumor adhesion to the endothelium. In a particular embodiment, the ability to increase the adhesion of tumor cells to the endothelium can be determined by means of an assay such as the one described in Example 2 with the appropriate modifications.

In a specific embodiment, said variant is a glycosylated mutant form of the protein comprising the amino acid sequence shown in SEQ ID NO: 1, which maintains the ability to increase the adhesion of tumor cells to the endothelium. Said mutant form may have insertions, deletions or modifications of one or more amino acids with respect to the protein comprised in SEQ ID NO: 1, on the proviso that it contains at least one residue of a sugar residue in any of the potential glycosylation sites and conserves the ability to increase the adhesion of tumor cells to the endothelium.

Likewise, as it is used in this description, the term "fragment" relates to a peptide comprising a portion of said protein comprising the amino acid sequence SEQ ID NO: 1, i.e., a contiguous amino acid sequence comprised within said SEQ ID NO: 1, which contains at least one sugar residue in any of the potential glycosylation sites; for use thereof in this inventive aspect of which it is glycosylated (i.e., it contains at least one sugar residue in any of the potential glycosylation sites) and is functionally equivalent, i.e., it maintains the ability to increase the adhesion of tumor cells to the endothelium.

The glycosylated protein of the invention, as well as the non-glycosylated protein of the invention, at least has the ability to increase the adhesion of tumor cells to the endothelium, which ability can be determined by contacting the tumor cells with endothelial cells stimulated with said glycosylated protein of the invention by means of the binding or interaction of said glycosylated protein of the invention with the suitable receptor, the activation of which seems to stimulate the tumor adhesion to the endothelium. The glycosylated protein of the invention can further have the ability to bind to a suitable receptor, such as a cytokine receptor (thus mimicking the corresponding cytokine) or a receptor to which or with which the *C. albicans* Kre9 protein binds or interacts; some of said receptors can stimulate cell adhesion to the endothelium. In a particular embodiment, the receptor to which the glycosylated protein of the invention binds is a cytokine receptor, such as the IL-1β receptor, so said glycosylated protein of the invention can act by mimicking said cytokine (IL-1β). Therefore, the glycosylated protein of the invention can be used in the identification of compounds potentially useful in the prevention and/or treatment of diseases associated with unwanted cell adhesion. The glycosylated protein of the invention can also have the ability to induce an inflammatory response, and therefore, due to its proinflammatory potential, act as an antigen, so it could be used in the prevention and/or treatment of a disease caused by an infectious agent.

If desired, the glycosylated protein of the invention can be part of a fusion protein. In this sense, by way of nonlimiting illustration, said fusion protein can contain a region A formed by a first peptide comprising the glycosylated protein of the invention bound to a region B comprising a second peptide. Said second peptide can be any suitable peptide, for example, a peptide useful for identifying the recombinant protein and/or for purifying the recombinant protein. Said region B can be bound to the amino-terminal region of said region A, or alternatively, said region B can be bound to the carboxyl-terminal region of said region A. Both regions A and B can be bound directly or through a spacer peptide (linker) between said regions A and B. The fusion protein can be obtained by conventional methods known by the persons skilled in the art, for example, by means of the gene expression of the nucleotide sequence encoding said fusion protein in suitable host cells.

The glycosylated protein of the invention can be obtained from an organism producing said glycosylated protein by means of a process which comprises cultivating said organism under conditions suitable for the expression of said protein and recovering it. In a particular embodiment of this invention, the organism producing the glycosylated protein of the invention is a fungus or yeast, or alternatively, a eukaryotic cell comprising a polynucleotide encoding the glycosylated protein of the invention; in a particular embodiment, said organism is a species of the genus *Candida,* e.g., *C. albicans.*

The nucleic acid sequence encoding said protein can be identified and isolated from the information provided by the glycosylated protein of the invention by means of using conventional techniques known by the persons skilled in the art, for example, by means of creating genomic DNA libraries (DNAg) or libraries of complementary DNA (cDNA) of organisms producing said protein; the design of oligonucleotides suitable for amplification by means of polymerase chain reaction (PCR), a region of the genomic clone of the organisms producing said protein which serves for obtaining probes intended for scrutinizing said genomic libraries; and analyzing and selecting the positive clones. In a particular embodiment, a pair of oligonucleotide primers consisting of the oligonucleotides the sequences of which are shown in SEQ ID NO. 3 and SEQ ID NO: 4, has been designed to amplify the gene encoding the *C. albicans* Kre9 protein. Said oligonucleotide primers are an additional aspect of this invention.

In a particular embodiment, the polynucleotide encoding the glycosylated protein of the invention comprises or consists of the nucleotide sequence shown in SEQ ID NO: 2. In a specific embodiment, the polynucleotide encoding the native (glycosylated) *C. albicans* Kre9 protein has the amino acid sequence shown in SEQ ID NO: 2 [Accession: XM_717838; Version: XM_717838.1, GI: 68465725; organism *C. albicans* SC5314]. In another specific embodiment, the polynucleotide encoding the glycosylated *C. albicans* Kre9 protein has the nucleotide sequence described in US 6,582,911 B1 (specifically in SEQ ID NO: 1 included in said United States patent).

Alternatively, the polynucleotide encoding the glycosylated protein of the invention may have variations in its sequence with respect to the nucleotide sequence shown in SEQ ID NO: 2, for example, substitutions, insertions and/or deletions of one or more nucleotides, on the proviso that the resulting polynucleotide encodes a non-glycosylated protein of the invention. Therefore, polynucleotides within the scope of the present invention are substantially homologous to the polynucleotide the nucleotide sequence of which is shown in the SEQ ID NO: 2 and encodes a non-glycosylated protein of the invention.

Said polynucleotide encoding the glycosylated protein of the invention can be contained in a gene construct comprising said polynucleotide, which can incorporate, operatively bound thereto, a regulatory sequence for the expression of said polynucleotide encoding the glycosylated protein of the invention, thus forming an expression cassette. Likewise, said gene construct can be inserted in a suitable vector. The choice of the vector will depend on the host organism in which said vector is going to be subsequently introduced. In a particular embodiment, the vector is a plasmid useful for transforming cells or eukaryotic organisms, e.g., *S. cerevisiae.* Said vector can be used for transforming organisms susceptible of being transformed by said vector. Given that the protein to be expressed (glycosylated protein of the invention) must be glycosylated, said organisms are eukaryotic organisms, for example, yeasts such as *S. cerevisiae* (US 6,582,911), etc.

In another aspect, the invention relates to a process for obtaining a glycosylated protein of the invention, which comprises cultivating a eukaryotic organism containing the sequence encoding the glycosylated protein of the invention under conditions which allow producing said protein and, if desired, recovering said glycosylated protein of the invention from the culture medium. The conditions for optimizing the cultivation of said eukaryotic organism of the invention will depend on the eukaryotic organism used; said conditions are known by the persons skilled in the art. The process for producing the glycosylated protein of the invention optionally includes isolating and purifying said glycosylated protein of the invention.

As has been previously mentioned, the glycosylated protein of the invention has the ability to increase the adhesion of tumor cells to the endothelium and the ability to bind to a suitable receptor, such as a cytokine receptor (thus mimicking the corresponding cytokine) or a receptor to which or with which the *C. albicans* Kre9 protein binds or interacts, for example, receptors that can stimulate cell adhesion to the endothelium, such as the IL-1β receptor, so said non-glycosylated protein of the invention can act by mimicking said cytokine (IL-1β). Therefore, the glycosylated protein of the invention can be used in the identification of compounds potentially useful in the prevention and/or treatment of a disease associated with unwanted cell adhesion.

The term "disease associated with unwanted cell adhesion" has been previously defined and includes cancer, metastasis, restenosis, diseases which stimulate a cellular immune response and inflammatory diseases.

Identification of potentially therapeutic compounds

Therefore, in another aspect, the invention relates to an in vitro method for identifying a compound potentially useful for the prevention and/or treatment of a disease associated with unwanted cell adhesion, hereinafter method for identifying (II) potentially therapeutic compounds of the invention, which comprises:

a) contacting a cell which expresses a receptor to which or with which a glycosylated protein of the invention binds or interacts with a glycosylated protein of the invention and with the candidate compound, and

b) identifying those compounds which inhibit the binding of said glycosylated protein of the invention to said receptor.

In a particular embodiment, the method for identifying potentially therapeutic compounds of the invention is particularly useful and suitable for identifying and selecting compounds potentially useful for the prevention and/or treatment of tumor processes, metastatic processes, restenosis, diseases which stimulate a cellular immune response and/or inflammatory diseases.

Although virtually any cell which expresses a receptor to which or with which a glycosylated protein of the invention binds or interacts can be used in the implementation of said method, in a particular embodiment, said cell which expresses a receptor to which or with which a glycosylated protein of the invention binds or interacts is a cell expressing a cytokine receptor, e.g., an IL-1β receptor, or a receptor of any other cytokine, or generally a cell expressing a receptor to which the *C. albicans* Kre9 protein binds; thus, in a particular embodiment, said cell which expresses a receptor to which or with which a glycosylated protein of the invention binds or interacts is a suitable cell from the endothelium, for example, a cell from the hepatic sinusoidal endothelium (HSE).

Treatment and/or prevention of diseases associated with unwanted cell adhesion

The compounds identified according to the method for identifying potentially therapeutic compounds of the invention can be used in the prevention and/or treatment of a disease associated with unwanted cell adhesion. Therefore, in another aspect, the invention relates to an inhibitory agent of the activity of the glycosylated protein of the invention for the prevention and/or treatment of a disease associated with unwanted cell adhesion, such as a tumor process, a metastatic process, restenosis, a disease which stimulates a cellular immune response and/or an inflammatory disease. In other words, in another aspect, the invention relates to the use of an inhibitory agent of the activity of the glycosylated protein of the invention in the manufacture of a pharmaceutical composition for the prevention and/or treatment of a disease associated with unwanted cell adhesion, such as a tumor process, a metastatic process, restenosis, a disease which stimulates a cellular immune response and/or an inflammatory disease.

In another aspect, the invention relates to a method for the prevention and/or treatment (II) of a disease associated with unwanted cell adhesion, such as a tumor process, a metastatic process, restenosis, a disease which stimulates a cellular immune response and/or an inflammatory disease, which comprises administrating a therapeutically effective amount of an inhibitory agent of the activity of a glycosylated protein of the invention (occasionally referred to as "inhibitory agent (II) of the invention") to a subject in need of treatment

In the context of the present invention, the term "glycosylated protein of the invention" has already been previously defined. In a particular embodiment of the invention, the glycosylated protein of the invention comprises or consists of the amino acid sequence shown in SEQ ID NO: 1, or the native (glycosylated) Candida spp. Kre9 protein, e.g., the glycosylated *C. albicans* Kre9 protein, such as the glycosylated *C. albicans* Kre9 protein having the amino acid sequence shown in SEQ ID NO: 2 of United States patent US 6,582,911 B1. In another particular embodiment of the invention, the glycosylated protein of the invention is a functionally equivalent variant or fragment of the aforementioned non-glycosylated proteins.

In the context of the present invention, "inhibitory agent (II) of the invention" is understood as any substance or compound capable of preventing or blocking the glycosylated protein of the invention from performing its function (activity), i.e., preventing said glycosylated protein of the invention from activating/stimulating the adhesion of tumor cells to the endothelium. Said inhibitory agent (II) of the invention can be the same as or different from said inhibitory agent (I) of the invention. Although it is not intended to be bound by any theory, it is believed that said activity for stimulating tumor adhesion to the endothelium can be performed by means of the glycosylated protein of the invention activating the suitable receptors (e.g., the IL-1β receptor, etc.), so the binding of a compound to said glycosylated protein of the invention can result in the glycosylated protein of the invention not being able to bind to the receptor or receptors thereof. Assays to determine whether a compound is an inhibitory agent of a glycosylated protein of the invention can be designed by persons skilled in the art from the information provided by this invention, for example, from the information contained in Example 2.

By way of non-limiting illustration, inhibitory agents of the activity of the non-glycosylated protein of the invention for use thereof in the present invention include, for example, inhibitory peptides of said glycosylated protein of the invention, antibodies directed specifically against epitopes of said glycosylated protein of the invention which are essential for carrying out the function thereof, etc. Therefore, in a particular embodiment of the invention, said inhibitory agent of the invention is selected from the group consisting of inhibitory peptides and antibodies.

As it is used in this inventive aspect, the term "inhibitory peptide" relates to a peptide capable of binding to a glycosylated protein of the invention and inhibiting its activity such as hereinbefore explained, i.e., preventing the glycosylated protein of the invention from being able to induce or stimulate the adhesion of tumor cells to the endothelium.

In the context of this inventive aspect of the present invention, "inhibitory antibody" is understood as any antibody capable of binding to a glycosylated protein of the invention, preventing said glycosylated protein of the invention from being able to induce or stimulate the adhesion of tumor cells to the endothelium. Said antibodies can be prepared by using any of the methods that are known by the person skilled in the art and which have been mentioned above, but in this case using a glycosylated protein of the invention instead of a non-glycosylated protein of the invention.

In a particular embodiment, said inhibitory antibody is an antibody which specifically recognizes a glycosylated protein of the invention, such as a glycosylated protein comprising or consisting of the amino acid sequence shown in SEQ ID NO: 1, or the glycosylated Candida spp. Kre9 protein, e.g., the glycosylated *C. albicans* Kre9 protein, such as the glycosylated *C. albicans* Kre9 protein having the amino acid sequence shown in SEQ ID NO: 2 of United States patent US 6,582,911 B1. In another particular embodiment, said inhibitory antibody is an antibody which specifically recognizes a functionally equivalent variant or fragment of the aforementioned glycosylated proteins. In a particular embodiment, said inhibitory antibody is an antibody which recognizes IL-1β, e.g., an anti-mammal IL-1β antibody.

As described at the beginning of the description, a new therapeutic window in the treatment of diseases associated with unwanted cell adhesion, such as tumor processes (cancer), metastatic processes (metastasis), restenosis, diseases which stimulate a cellular immune response and/or inflammatory diseases, has now been discovered.

Therefore, in another aspect, the invention relates to a pharmaceutical composition, hereinafter "pharmaceutical composition C of the invention", comprising a therapeutically effective amount of an inhibitory agent (II) of the invention together with a pharmaceutically acceptable carrier for the treatment of diseases associated with unwanted cell adhesion. Examples of diseases associated with unwanted cell adhesion include tumor processes (cancer), metastatic processes (metastasis), restenosis, diseases which stimulate a cellular immune response and/or inflammatory diseases and have been mentioned previously in the present description.

The terms "therapeutically effective amount", "treatment" or "treating", "vehicle, adjuvant and/or carrier" and "pharmaceutically acceptable" have been previously defined in this description.

The inhibitory agent (II) of the invention as well as the pharmaceutical compositions containing it can be used together with other additional drugs useful in the treatment of diseases associated with unwanted cell proliferation and/or adhesion. Said additional drugs can be part of pharmaceutical composition C of the invention, or they can alternatively be provided in the form of a separate pharmaceutical composition for the simultaneous or non-simultaneous administration thereof with respect to that of pharmaceutical composition C of the invention comprising said inhibitory agent (II) of the activity of a glycosylated protein of the invention. Examples of said additional drugs useful in the treatment of diseases associated with unwanted cell proliferation have been mentioned previously.

Induction of an inflammatory response

Alternatively, the inhibitory agent (II) of the invention as well as the pharmaceutical compositions containing it can be used together with other additional drugs useful in the treatment of fungal infections, for example, antifungal agents useful in the prevention and/or treatment of candidiasis. Said additional drugs can be part of pharmaceutical composition C of the invention, or they can alternatively be provided in the form of a separate composition for the simultaneous or non-simultaneous administration thereof with respect to that of pharmaceutical composition A of the invention comprising said inhibitory agent of the activity of a glycosylated protein of the invention. Examples of drugs useful in the treatment of fungal infections have been previously mentioned.

Pharmaceutical composition C of the invention can be administered by any suitable administration route, for example, orally, parenterally (for example, subcutaneously, intraperitoneally, intravenously, intramuscularly, etc.), rectally, etc.

Illustrative examples of the different pharmaceutical dosage forms and the manufacture thereof have been previously described in this description.

Likewise, as previously mentioned, the glycosylated protein of the invention may have the ability to induce an inflammatory response, so, due to its proinflammatory potential, said glycosylated protein of the invention can act as an antigen, and could be used as such in the prevention and/or treatment of a disease caused by an infectious agent. Although it is not intended to be bound by any theory, it is believed that the native *C. albicans* Kre9 protein, a representative example of a glycosylated protein of the invention, presumably acts by binding to the IL-1β receptor, so it has a higher proinflammatory potential due to its ability to bind to and activate said receptor, acting as if it is was endogenous IL-1β, one of the most important proinflammatory cytokines in the immune response.

Therefore, in another aspect, the invention relates to a pharmaceutical composition, hereinafter "pharmaceutical composition D of the invention", comprising a therapeutically effective amount of a glycosylated protein of the invention together with a pharmaceutically acceptable carrier for the prevention and/or treatment of a disease caused by an infectious agent.

The term "disease caused by an infectious agent" has been previously defined in this description and it relates to the clinical manifestation resulting from an infection stimulated by microorganisms, e.g., bacteria, fungi, viruses, protozoa, etc., or by prions. In a particular embodiment, said infectious agent is an infectious agent which expresses the *C. albicans* Kre9 protein or a protein similar to said *C. albicans* Kre9 protein which may influence the inflammatory response, for example, a species of the genus Candida, etc. Likewise, in another particular embodiment, said infectious agent is a microorganism which can be spread through the blood stream and adhere itself to organs activating an inflammatory and immune response. In a specific embodiment, said disease caused by an infectious agent is candidiasis.

In a particular embodiment, said non-glycosylated protein of the invention present in pharmaceutical composition D of the invention is a glycosylated protein comprising or consisting of the amino acid sequence shown in SEQ ID NO: 1, or the native (glycosylated) Candida spp. Kre9 protein, e.g., the glycosylated *C. albicans* Kre9 protein, such as the glycosylated *C. albicans* Kre9 protein having the amino acid sequence shown in SEQ ID NO: 2 of United States patent US 6,582,911 B1. In another particular embodiment, said non-glycosylated protein of the invention is a functionally equivalent variant or fragment of the aforementioned glycosylated proteins.

The glycosylated protein of the invention as well as the pharmaceutical compositions containing it can be used together with other additional drugs useful in the treatment of diseases caused by one or more infectious agents. Said additional drugs can be part of pharmaceutical composition D of the invention, or they can alternatively be provided in the form of a separate pharmaceutical composition for the simultaneous or non-simultaneous administration thereof with respect to that of pharmaceutical composition D of the invention comprising said glycosylated protein of the invention. Illustrative examples of other additional drugs useful in the treatment of diseases caused by infectious agents have been previously mentioned in this description.

In another aspect, the invention relates to a glycosylated protein of the invention for the prevention and/or treatment of a disease caused by an infectious agent; i.e., in another aspect, the invention relates to the use of a glycosylated protein of the invention in the manufacture of a pharmaceutical composition for the prevention and/or treatment of a disease caused by an infectious agent. In a particular embodiment, said disease caused by an infectious agent is a disease caused by microorganisms, e.g., bacteria, fungi, viruses, protozoa, etc., or by prions. In a particular embodiment, said infectious agent is an infectious agent which expresses the *C. albicans* Kre9 protein or a protein similar to said *C. albicans* Kre9 protein that may influence the inflammatory response, for example, a species of the genus *Candida,* a related fungus which may be spread through the blood, etc. Likewise, in another particular embodiment, said infectious agent is a microorganism that can be spread through the blood stream and adhere itself to organs activating an inflammatory and immune response. In a specific embodiment, said disease caused by an infectious agent is candidiasis.

In a particular embodiment, said glycosylated protein of the invention present in pharmaceutical composition D of the invention is a glycosylated protein comprising or consisting of the amino acid sequence shown in SEQ ID NO: 1, or the glycosylated *Candida* spp. Kre9 protein, e.g., the non-glycosylated *C. albicans* Kre9 protein, such as the glycosylated *C. albicans* Kre9 protein having the amino acid sequence shown in SEQ ID NO: 2 of United States patent US 6,582,911 B1. In another particular embodiment, said non-glycosylated protein of the invention is a functionally equivalent variant or fragment of the aforementioned glycosylated proteins.

In another aspect, the invention relates to a method for the prevention and/or treatment of a disease caused by an infectious agent, which comprises administrating a therapeutically effective amount of a glycosylated protein of the invention to a subject in need of treatment.

In another aspect, the invention relates to the use of a kit comprising:

an inhibitory agent (II) of the invention (for example, a compound capable of binding to a glycosylated protein of the invention and blocking the binding of said protein to its receptor or receptors present in endothelial cells) for the prevention and/or treatment of a disease associated with unwanted cell adhesion,

or alternatively,

a glycosylated protein of the invention for the prevention and/or treatment of a disease caused by an infectious agent.

Said "kit" has the characteristics previously mentioned in this description with respect to components, instructions for simultaneous, sequential or separate administration of the different pharmaceutical formulations present in the kit, etc. All the particular embodiments which define this inventive aspect have been mentioned, described and explained in previous inventive aspects, therefore it is not necessary to repeat them.

The following examples illustrate the invention and should not be considered as limiting of the scope thereof.

EXAMPLE 1

Expression of the recombinant *C. albicans* Kre9 (CaKre9) protein in *E. coli*

1. Materials and methods

1.1 Yeasts

Four strains of *Candida albicans,* specifically, the UPV1413 *C. albicans* and UPV1360 *C. albicans* strains belonging to the Type Culture Collection of the Universidad del Pais Vasco (UPV/EHU) which were isolated from patients with systemic candidiasis, the CA2 strain, an agerminative mutant kindly provided by A Cassone (Istituto Superiore di Sanità, Rome, Italy) and the NCPF 3153 reference strain from the National Collection of Pathogenic Fungi (NCPF) (Bristol, United Kingdom), have been used.

The strains were maintained at 4°C in inclined tubes of Sabouraud glucose agar (SDA) [1 liter of Sabouraud broth (SDB) (20 g/l glucose and 10 g/l peptone) and 15 g/l of agar] after a prior incubation of 24 hours at 24°C. Monthly reseeding was performed in the same maintenance medium.

1.2 Extraction of yeast DNA

The yeasts (UPV1413 *C. albicans*) were cultivated in 20 ml of Sabouraud broth (SDB) [20 g/l glucose and 10 g/l peptone] for 24 hours. The cells obtained were collected by means of centrifugation and were washed twice with sterile distilled water. The yeasts were subsequently resuspended in 400 $\mu$l of 10 mM pH 8 Tris buffer and the cell suspension was passed into a microcentrifuge tube with 0.3 g of sterile glass beads (0.5 mm in diameter). The tubes were vigorously stirred for 1 minute in a vortex. The mixture was incubated for 10 minutes in boiling water, was centrifuged at 2,500 g, the supernatant was collected and DNA concentration was measured.

1.3 Measurement of the DNA concentration

The measurement of the DNA concentration in a sample that is the product of genomic or plasmid DNA extraction or a PCR product was performed by means of measuring the absorbance at 260/280 nm in a NanoPhotometer (Implen).

1.4 Cloning DNA into plasmid vectors

The basic techniques for handling nucleic acids have been described in detail by Sambrook *et al.* (Sambrook *et al.* 2001, Molecular cloning: A laboratory manual).

The vector used in the cloning and expression experiments of the recombinant *C. albicans* Kre9 (CaKre9) protein was the commercial pET-Blue (Novagen) vector. Among the interesting particularities that this vector has is the presence of a sequence encoding the amino-terminal end for an HSV tag which allows immunodetection of the recombinant protein provided that the insert has been cloned in the correct reading frame. Likewise, the pET-Blue vector has a sequence encoding a 6 histidine tag, which allows affinity purification in a column of the recombinant protein provided that, such as in the case of the HSV tag, the insert has been cloned in the correct reading frame. This vector further has the characteristic that its multiple cloning are where the restriction enzymes cut a single time in the entire plasmid, is located within the gene encoding the lacZ-$\alpha$ peptide [the plated colonies in the presence of isopropyl-beta-D-1-thiogalactopyranoside (IPTG) and X-gal are thus blue whereas when the insert is introduced, the lacZ-$\alpha$ peptide is truncated and the colonies formed are white].

1.5 Obtaining the DNA insert

The DNA sequence to be cloned into the pET-Blue vector was a PCR (polymerase chain reaction) product obtained from the amplification of the coding sequence of the of *C. albicans* KRE9 (CaKre9) gene from the genomic DNA once the absence of introns is confirmed. The primers used for producing the recombinant CaKre9 protein were the following:

Forward: 5'-aaccg**gaattc**gctatcagacacatctagcaaa-3' (SEQ ID NO: 3); and
Reverse: 5'-tcctct**ctgcag**atccaaccatcttcttctc-3' (SEQ ID NO: 4)

The reaction mixtures used in the PCR were formed by 3.5 $\mu$l of water, 2.5 $\mu$l of 10X NH4 buffer, 1.5 $\mu$l of 50 mM $MgCl_2$, 0.75 $\mu$l of 25 mM dNTPs (dATP, dCTP, dGTP and dTTP), 10 $\mu$l (860 ng) of yeast DNA (1/10), 3 + 3 $\mu$l of primers and 0.75 $\mu$l of Taq polymerase (Bioline).
The protocol followed for amplifying the *C. albicans* gene encoding the Kre9 (CaKre9) protein by means of PCR was the following:

| Temperature (°C) | Time (minutes) | Cycles |
|---|---|---|
| 95 | 5 | 2 |
| 95 | 1 | 40 |
| 48 | 1 | |
| 72 | 1 | |
| 72 | 10 | 1 |

While designing the primers, specific target sequences were introduced for the restriction enzymes and more bases were added at the end to facilitate the work of the enzymes.
In order to visualize the amplified sequences, the DNA bands were separated by means of agarose gel electrophoresis at the concentration of 1.5% and 0.5 $\mu$g/ml of ethidium bromide, at 100 V for approximately 45 minutes in a Wide Mini-Sub Cll GT (Bio-Rad) electrophoresis chamber. When needed, the DNA was recovered from the gel using for that purpose the Ultra Clean GelSpin Gel purification kit (MoBio) following the instructions described by the manufacturer.

1.6 Digestion with restriction endonucleases and ligation

The conditions for using the restriction enzymes were as specified by the commercial supplier (Takara). The concentrations indicated below were mixed in a 500 $\mu$l microtube:

| Reaction mixture for cutting the pET-Blue plasmid with restriction enzymes | |
|---|---|
| $H_2O$ | 10 $\mu$l |
| Buffer H (10X) | 2 $\mu$l |
| EcoRI | 1 $\mu$l (15 u) |
| PstI | 1 $\mu$l (15 u) |
| Plasmid | 6 $\mu$l (150 ng) |

| Reaction mixture for cutting the *C. albicans* KRE9 (CaKre9) gene amplified by means of PCR with restriction enzymes | |
|---|---|
| $H_2O$ | 40 $\mu$l (600 ng) |
| Buffer H (10X) | 5 $\mu$l |
| EcoRI | 2.5 $\mu$l (15 u) |
| PstI | 2.5 $\mu$l (15 u) |

The incubation was performed at 37°C for 1 hour.

The cut DNA, obtained after digestion with restriction enzymes, was purified before proceeding to ligation by means of the Ultra Clean GelSpin Gel purification kit (MoBio), using the T4-DNA ligase enzyme following the instructions described by the manufacturer. In the ligation experiments, the ratio of vector molar:insert used was 1:3. The ligation reaction was carried out at 16°C for approximately 18 hours.

1.7 Transformation of competent cells

Novablue Singles (Novagen) competent cells were used for the transformation experiments, assuring high transformation efficiency and a higher number of plasmid copies. The vials of competent cells maintained at -80°C were thawed by maintaining them on ice for approximately 5 minutes, after which 10 ng of ligation mixture were added. The tubes were incubated 5 more minutes on ice, and the cells were then subjected to a heat shock for 1 minute at 42°C and were incubated 5 more minutes on ice. They were then seeded in LB agar plates supplemented with 12.5 $\mu$g/ml tetracycline and 50 $\mu$g/ml carbenicillin, and were incubated overnight at 37°C.

1.8 Rapid screening of positive clones

The technique described by Le-Gouill & Dery [Le Gouill, C. & Déry, C.V. Nucleic Acids Research. 19(23):6655, 1991] was followed. This process allowed rapid screening of plasmid DNA from the colonies grown in agar plates without the need for prior extraction. The surface of the grown colony was touched with sterile tips to reseed it in another agar plate supplemented with the corresponding antibiotic to thus obtain a replica of the colony. The rest of the colony was transferred to a tube with 10 $\mu$l of lysis buffer [90 $\mu$l of loading buffer (4 g of sucrose, traces of bromophenol blue and 10 ml of Mili Q water), 400 $\mu$l of buffer I (100 $\mu$l of 10% SDS, 20 $\mu$l of 10 N NaOH and 880 $\mu$l of MiliQ water) and 110 $\mu$l of MiliQ water] and was resuspended by carefully pipetting to avoid producing bubbles. 3 $\mu$l of buffer II [500 $\mu$l of potassium acetate, 78 $\mu$l of 85% formic acid and 222 $\mu$l of MiliQ water] were added and the tubes were centrifuged for 4 minutes at 13,000 rpm; the samples were loaded in a 0.7% agarose gel to avoid precipitation.

1.9 Purification of plasmid DNA

The NucleoSpin Plasmid (Macherey-Nagel) kit was used for preparing the plasmid DNA from a liquid culture of transformed cells. A sample was digested with the restriction enzymes used in cloning and was separated by means of agarose gel electrophoresis after digestion to confirm the presence of the insert in the obtained DNA.

1.10 Induction of the expression of recombinant proteins

After confirming the presence of the insert of interest in the vector and insertion thereof in the correct reading frame with the His and HSV tags, the Tuner (DE3)pLac (Novagen) cells were transformed with the recombinant plasmid. The methodology followed for the transformation of this strain was the same as the one followed in the case of the Novablue Singles strain. In this case, the cells were seeded in LB agar plates supplemented with 50 $\mu$g/ml carbenicillin and 34 $\mu$g/ml chloramphenicol.

5 ml of LB broth [10 g/l of tryptone, 5 g/l of yeast extract and 5 g/l of sodium chloride] supplemented with carbenicillin and chloramphenicol from a colony transformed with the recombinant plasmid were inoculated. A colony of cells transformed with the original plasmid was used as control. The tubes were incubated overnight at 37°C and 120 rpm. 500 $\mu$l of the culture were taken the following day to inoculate 4.5 ml of fresh medium and they were inoculated until reaching optical density at 600 nm ($OD_{600}$) between 0.5-1. Once said optical density is reached, the culture was separated into 2 aliquots of 2.5 ml and a final concentration of 0.5 mM IPTG was added to one of the aliquots. The cultures were incubated 4 more hours, after which 1 ml of each culture was centrifuged. The supernatant was discarded and the precipitate was resuspended in 200 $\mu$l of electrophoresis buffer and was analyzed by means of electrophoresis in 10% SDS-PAGE gel.

1.11 Purification of the recombinant proteins

The purification of the recombinant proteins was performed by using the His-Bind (Novagen) kit. Briefly, a culture in LB medium supplemented with carbenicillin and chloramphenicol grown for 12 hours was used to inoculate 250 ml of fresh medium and to induce protein expression as described in section 1.10. The cells were collected by centrifugation at 2,500 g for 5 minutes, then dried to the maximum and weighed. For the purpose of increasing the extraction efficiency, the cell pellet was maintained overnight at -20°C. On the following day, 5 ml of the BugBuster reagent were added per gram of cells and 1 $\mu$l of benzonase was added per milliliter of BugBuster reagent used to resuspend the cells. The mixture was incubated at room temperature in an orbital shaker for 20 minutes, after which the tubes were centrifuged at 13,000 rpm for 20 minutes at 4°C. The proteins contained in the soluble fraction were thus separated from the cell residues. The supernatants were maintained at -20°C until the use thereof.

The purification by means of affinity chromatography by using the His-Bind resin is a rapid protein purification method having a 6 histidine tag (6 His) at room temperature or, alternatively, at 4°C. The manufacture of the column was performed by following the protocol recommended by the supplier. 1 g of cell extract which was finally eluted was treated in 5 ml of elution buffer in each purification batch. The degree of purification obtained and the yield were analyzed by means of SDS-PAGE gel electrophoresis and staining with Coomassie blue and immunodetection of the recombinant protein with the HSV-Tag (Novagen) monoclonal antibody.

### 1.12 SDS-PAGE (1D)

The different protein extracts were analyzed by means of SDS-PAGE in the Miniprotean II (Bio-Rad) system for 45 minutes at 70 mA, 100 W and 200 V. The high molecular weight HMW standards (Sigma) [Myosin (205 kDa), $\alpha$-galactosidase (116 kDa), phosphorylase B (97.4 kDa), bovine albumin (66 kDa), ovoalbumin (45 kDa) and carbonic anhydrase (29 kDa)] were use as molecular weight markers for determining the molecular weights of the bands obtained in electrophoresis.

### 1.13 Coomassie G250 Staining

Three washes in distilled water lasting 5 minutes were performed prior to adding the Bio-Safe dye (Bio-Rad) based on colloidal Coomassie blue G250. The dye was then added for 1 hour. It was again washed with distilled when this time ended.

### 1.14 Western Blot

Immobilon-P membranes (Millipore) previously treated in an organic solvent (methanol) and subsequently treated in deionized water were used. Briefly, the membrane previously wet with methanol and miliQ water was maintained for 10 minutes in TBS buffer [50 mM Tris-HCl (pH 7.5) and 150 mM NaCl] before the incubations start; the free radicals were then blocked with TBSL buffer [100 ml of TBS buffer and 5 g of skim milk] for 1 hour at 37°C while stirring. After that, incubation was carried out with the primary antibody, anti-mouse IL-1$\beta$ (R&D Systems), in a 1/500 dilution in TBSL buffer for 1 hour at 37°C. Subsequently, 3 washes with TBS buffer lasting 5 minutes were performed and the incubation with the secondary anti-IgG antibody labeled with diluted peroxidase (anti-IgG-HRP) 1/20,000 in TBSL buffer was carried out for 1 hour at room temperature. Finally, the membranes were washed three times for 10 minutes with TBS buffer and the reaction was visualized by means of Immobilon Western Chemiluminescence reagent (Millipore) on Curix RP-2 (Kodak) film sheets.

### 1.15 Computer analysis of images

The computer analysis of the electrophoresis of the different purified fractions was performed with the Image-Master 2D Platinum (GE Healthcare) computer program from the gels stained with Coomassie blue G250, with silver staining or developing with chemiluminescence.

### 2. Results

### 2.1 Obtaining the recombinant CaKre9 protein

Due to the strategy followed for producing and expressing the recombinant *C. albicans* Kre9 protein [recombinant CaKre9] based on the use of pET-Blue plasmid and *E. coli,* said recombinant CaKre9 protein was obtained in the form of a fusion protein comprising the amino acid sequence of the *C. albicans* Kre9 (CaKre9) protein fused to an HSV tag and 6 histidine residues [(His)$_6$]; therefore, the use of the terms "recombinant CaKre9 protein" and "CaKre9-(His)$_6$ fusion protein" in the examples to refer to the recombinant protein produced is equivalent.

The nucleotide sequence of the CaKre9 protein that was amplified consisted of 834 bp. The cloning and expression vector was the commercial pET-Blue (Novagen) plasmid. The nucleotide sequence of said CaKre9 protein was amplified by means of the designed primers which contained the restriction sites for EcoRI and PstI. The construct was called pET-KRE9. Said nucleotide sequence of the CaKre9 protein was introduced into the plasmid lacking the reading frame thereof, whereby the recombinant protein was expressed with the HSV-His tag (Figure 1).

After the transformation of the Novablue cells with the pET-KRE9 construct and for detecting the positive clones, a rapid screening of the colonies which appeared in white or blue was performed. Three of the clones taken as positive were chosen to make a manufacture of plasmid DNA and to be digested with its respective restriction enzymes for confirming the insert size.

These clones were selected and the recombinant plasmids were sequenced to determine if the insert had been cloned into the correct reading frame and if the sequence thereof was the desired sequence. Sequencing was performed in the Genomic Department of the UPV/EHU.

Once the size and the sequence of the insert were confirmed, the transformation of the Tuner (Novagen) cells with one of the selected clones was carried out. The protein expression of the induced and non-induced recombinant strain was analyzed by means of SDS-PAGE gels. The strain transformed with pET-Blue plasmid was used as negative control. Staining with Coomassie blue did not show the overexpression of any protein; however, the use of the anti-HSV antibody detected the presence of an approximately 45 kDa fusion protein (Figure 2).

After purification thereof by means of affinity chromatography in His Bind columns (Novagen) which retained the proteins having tags of between 6 and 10 histidines, the presence thereof was again confirmed by means of SDS-PAGE. As seen in Figure 2, the purification was satisfactory because the presence thereof could be confirmed by means of Coomassie blue staining and by means of Western Blot against the HSV antibody.

Finally, the recombinant CaKre9 protein was analyzed to see if it still had the capacity of being recognized by the anti-IL-1β antibody. The result shown in Figure 2 demonstrates that the fusion protein obtained (CaKre9) is also recognized by the anti-IL-1β antibody as the native *C. albicans* Kre9 protein. Therefore, the fusion protein obtained in *E. coli* from the *C. albicans* KRE9 (CaKre9) gene maintains the capacity of binding to the anti-IL-1β antibody blocking the function thereof.

3. Discussion

Previous studies performed by the inventors forming the subject-matter of the co-pending Spanish patent application No. P200900......., entitled "Composiciones farmacéuticas para el tratamiento de la metastasis" *(Pharmaceutical Compositions for the Treatment of Metastasis),* filed on 2 April 2009, in the name of Universidad del Pais Vasco and Pharmakine, S.L., have shown the importance of the IL-1β in the process initiated by the *C. albicans* mannoproteins, which induce an increase of tumor adhesion to the HSE and indicate the possible presence of a molecule capable of acting in place of IL-1β in the mannoprotein fraction. An anti- mouse IL-1β antibody was used for detecting a mannoprotein binding to the IL-1β receptor, activating it and further intensifying the response of the endothelium. This antibody produced in *E. coli* was generated against a recombinant mouse IL-1β capable of neutralizing the biological function of IL-1β, i.e., it binds to the protein part and prevents the molecule from binding to the receptor. Two-dimensional Western Blot of the mannoprotein fraction purified from *C. albicans* evaluated against the anti-IL-1β antibody and developed with chemiluminescence were performed. A not very intense but very specific reaction of an area of approximately 47-50 kDa and with an isoelectric point of 5.3, indicating the existence of a *C. albicans* mannoprotein that is recognized by an anti-IL-1β antibody in the mannoprotein fractions in which a higher increase of the tumor adhesion had been induced, was observed in the results of the immunodetection performed.

The identification of said mannoprotein could involve the identification of a *C. albicans* effector which stimulates the endothelium and, consequently, increases tumor adhesion. From the results obtained, the sequencing of the mannoprotein recognized by the anti-IL-1β antibody was carried out by means of LC-MS/MS (this technique allows identifying proteins through the sequencing of their peptides and allows analyzing complex mixtures of proteins without the need for prior fractioning). The results obtained by means of the LC-MS/MS sequencing indicate that the mannoprotein recognized by the anti-mouse IL-1β antibody is the *C. albicans* Kre9 protein. The *C. albicans* KRE9 gene encodes a protein similar to the product of the *S. cerevisiae* KRE9 gene. Its sequence consists of an ORF of 813 bp encoding a 29 kDa protein formed by 271 amino acids. The N-terminal hydrophobic region is a signal sequence approximately up to the point of binding between amino acids 21 and 22. It contains a characteristic of an O-glycosylation potential, typical of many wall mannoproteins. According to the Lussier group (Lussier et al., Proc Natl Acad Sci. 1998; 95:9825-9830) the disruption of the *C. albicans* KRE9 gene shows that KRE9 is required for the synthesis or assembly of the β-1,6-glucans. The homozygous mutant cells of *C. albicans* for the KRE9 gene grow poorly in galactose and are not capable of growing in filamentary form in serum and in media containing glucose. The gene is thus essential. In addition to these two reasons, said authors also indicate the product of the KRE9 gene as a candidate useful for being a specific target drug against fungi since the gene has not been found in the sequenced bacteria or archaebacterial genomes, it seems to be absent also in metazoa, and since the available data suggest that its location is on the cell surface, in which case a KRE9 inhibitor will not be needed to enter the fungal cell to be effective, thus further reducing the risk of resistant strains.

The cloning, expression and purification of *C. albicans* Kre9 (CaKre9) protein as a recombinant protein were carried out to effectively confirm that the *C. albicans* Kre9 (CaKre9) protein is involved in the stimulation of the endothelium which results in the increase of tumor adhesion thereto. The pET-Blue plasmid was chosen as the vector given the advantages it offers with regard to the fact that in addition to being an expression vector, it facilitates the selection of recombinant colonies with an insert through the system based on the lactose operon and that it has two consecutive sequences encoding an HSV tag for immunodetection and a polyhistidine tag for purification. The recombinant CaKre9 protein was cloned and overexpressed in *E. coli,* the protein with a molecular weight of approximately 45 kDa being detected by means of inmunoblotting with the anti-HSV antibody. The protein was purified by means of affinity chromatography in a column based on the histidines tag and the results after purification showed a correct purification process, after which a band that further maintains its capacity of binding to the anti IL-1β antibody can be visualized in 1D electrophoresis by means of Coomassie staining, as can be confirmed by performing inmunoblotting. Said purified recombinant CaKre9 protein was used to stimulate the endothelium and to observe its effect on tumor adhesion (Example 2).

EXAMPLE 2

Effect of the CaKre9 expressed in *E. coli* on tumor adhesion to the hepatic endothelium

1. Materials and Methods

1.1 Recombinant CaKre9 protein

The recombinant CaKre9 protein used in this assay, also occasionally identified as "CaKre9-$(His)_6$ fusion protein", was that obtained according to Example 1.

1.2 B16 melanoma cells

B16 melanoma (MB16) is a very differentiated malignant tumor which was isolated in Jackson Laboratories (Maine, USA) in 1954 from a spontaneous tumor developed in the skin of a mouse of the C57BL/6J strain. In 1972, starting from this line, IJ Fidler obtained cell variants with different metastatic potential by means of reiterated intravenous implants of the tumor cells obtained from the pulmonary metastatic focus. A line with high metastatic capacity known as B16F10 was thus selected (Fidler IJ. Nature. 1973; 242:148-149). This tumor was provided by the American Type Culture Collection (ATCC CRL 6323).

The B16 melanoma cells were cultivated in Dulbecco's Modified Eagle Medium (DMEM) supplemented with 10% fetal bovine serum (FBS), 100 units/ml of penicillin, and 100 μg/ml streptomycin. The cultures were maintained at 37°C in 5% $CO_2$ atmosphere.

1.3 HSE cells

The liver of anesthetized mice was perfused through the portal vein with Gey's Balanced Saline Solution (GBSS) (pH 7.4) at 1.5 ml/min for 3 minutes. The digestion of the tissue was then carried out by means of in situ perfusion with 10 ml of solution A (10 ml of 10 ml GBSS and 0.1% Pronase) followed by 15 ml of solution B (15 ml of GBSS, 0.083% (w/v) Collagenase and 0.033% (w/v) Pronase). The liver was then cut up and mixed in 13 ml of solution C (13 ml of GBSS, 0.038% Collagenase, 0.038% Pronase and 0.0005% (w/v) DNase I) at 37°C for 10 minutes. After this final enzymatic digestion the solution was diluted with GBSS and the entire volume was filtered through sterile nylon gauze. The filtrate was distributed in two 50 ml tubes and was centrifuged at 1,500 rpm for 7 minutes at 4°C. A second washing was performed in the same conditions. The cell suspension was centrifuged at 800 g for 15 minutes in metrizamide solution (2-(3-Acetamido-2,4,6-triiodo-5-(N-methylacetamido)benzamido)-2-deoxy-D-glucose) (10 ml of 10 ml GBSS and metrizamide at 17.5%). The cells located in the upper part of the gradient were then collected, resuspended in DMEM-HEPES (10 mM, pH 7.4) and centrifuged at 500 g for 7 minutes at 4°C. The cell pellet obtained was resuspended in a small volume of DMEM to calculate the ratio of viable cells by means of an exclusion test with Trypan blue and counting with hemocytometer. To obtain a population of sinusoidal cells in optimal conditions, they were seeded on a 1% type I collagen matrix. 24-well plates to which the collagen solution was added at the ratio of 1 ml/well were used for the manufacture of these culture plates with this matrix. After the incubation thereof at 37°C for at least 2 hours, the isolated cells were directly washed and seeded. The endothelial cells recently isolated in DMEM with 10% of FBS were added to the 24 well plates blocked with type I collagen at a ratio of $10^6$ cell/ml per well, and were washed after 2 hours. The resulting cultures of HSE adherent cells were considered pure as the other cell types were completely removed during the washes. The HSE cell cultures were stabilized and maintained in pyrogen-free DMEM medium supplemented with 10% fetal bovine serum, 100 units/ml of penicillin, and 100 μg/ml streptomycin at 37°C in a 5% $CO_2$ atmosphere for at least 12 hours. After culturing, the endothelial cells were washed and maintained in a medium without serum for at least 4 hours before performing any type of assay. In these conditions, the endothelial cells can be maintained in culture for a maximum 4-5 days before starting to lose their characteristics.

1.4 Tumor adhesion induced by the recombinant CaKre9 protein

The measurement of tumor adhesion induced by the recombinant CaKre9 protein expressed in *E. coli* was carried out by using the method based on fluorescence measurement. The MB16 cells were resuspended in a solution of 2',7'-bis-(2-carboxyethyl)-5,6-carboxyfluorescein acetoxymethyl ester (BCECF-AM) in DMEM-HEPES (40 $\mu$g/ml) and were incubated for 20 minutes at 37°C. The cells were then washed and resuspended in DMEM-HEPES at a concentration of 2 x $10^5$ cell/ml and the autofluorescence of the HSE culture was determined. The HSE cells were subsequently incubated with the recombinant CaKre9 protein at different concentrations (1, 5, 10, 50, 100, 200, 500, 1,000 and 2,000 ng/ml) for 8 hours. The non-treated HSE cells received basal medium as control. After extensive washing, the MB16 cells labeled with BCECF were added to the HSE cells culture (0.1 ml/well) and also to plastic or control wells covered with type I collagen.

Once the adhesion time elapsed, the co-cultures were excited with a light of 488 nm wavelength and the fluorescence emitted was registered at 530 nm by the BCEF incorporated to the tumor cells in a plate scanning fluorometer. Three vigorous washes of said co-cultures were then performed, removing the tumor cells that are not adhered to the endothelial cell monolayer . 1 ml of the same adhesion medium was then added in each well and the reading of the fluorescence emitted was carried out. A relative value (in arbitrary units of fluorescence intensity) with respect to the number of tumor cells added could thus be determined. The relative adhesion was calculated for each well according to the formula:

$$\texttt{Relative adhesion = (A - C)/(B - C)}$$

wherein
A is the fluorescence of the MB16 cells adhered to the HSE incubated with the recombinant CaKre9 protein;
B is the fluorescence of the MB16 cells adhered to the non-treated HSE cells; and
C is the fluorescence prior to the adhesion of tumor cells.

2. Results

An adhesion assay of tumor cells to the hepatic endothelium incubated with different concentrations of the recombinant CaKre9 protein [CaKre9-(His)6 fusion protein] was performed for the purpose of analyzing the effect of said fusion protein.

The results obtained are shown in Figure 3, which shows the effect of said recombinant CaKre9 protein on the increase of the adhesion of the B16M to the HSE. As seen in said Figure 3, the increase of the tumor adhesion induced by the recombinant CaKre9 protein varies depending on the concentration of protein used in a typical behavior that could be compared with the behavior of some cytokines. The recombinant CaKre9 protein induced an increase of more than 150% of the tumor adhesion (B16M cells) to the HSE with respect to the control at a concentration of 5 ng/ml. This effect involved the adhesion of a number of tumor cells occasionally close to 100% of the cells used in the assay. However, the effect decreased at higher concentrations.

Therefore, the increase of the tumor adhesion to the HSE stimulated by the recombinant CaKre9 protein was spectacular, furthermore taking into account that it is a fusion protein [KaCre9-(His)6] expressed in *E. coli,* which does not have any mannan adhered to said protein. This situation causes said fusion protein to be unable to stimulate the mannose receptor, and, consequently, trigger the cytokine cascade after stimulation thereof, which seems to corroborate the existence of a different inflammatory route than that which is stimulated by means of binding to the mannose receptor; said alternative route can be initiated in response to *C. albicans* proteins and also induces an increase of tumor adhesion to the hepatic endothelium. In this response, the recombinant CaKre9 protein is involved because it acts by increasing tumor adhesion to the endothelium, probably by mimicking the action of any proinflammatory cytokine such as IL-1$\beta$. This increase of tumor adhesion to the HSE incubated with the recombinant CaKre9 protein, which has no adhered carbohydrates, shows the importance of the protein part of the mannoproteins, demonstrating that not only the mannose receptor is involved in this process.

This assay also shows that the recombinant CaKre9 protein, such as the *C. albicans* Kre9 protein (CaKre9), acts by binding to the IL-1$\beta$ receptor and has a high proinflammatory potential due to the capacity of binding to and activating the IL-1$\beta$ receptor, acting as if it was endogenous IL-1$\beta$, one of the most important proinflammatory cytokines in the immune response.

3. Discussion

The purified recombinant CaKre9 protein (Example 1) was used to stimulate the endothelium and to observe the effect thereof in tumor adhesion. This assay unequivocally demonstrates the involvement of CaKre9 protein in the increase of the adhesion of tumor cells to the endothelium because a significant increase of the adhesion of tumor cells to the endothelium stimulated with recombinant CaKre9 with respect to the non-stimulated endothelium is induced. Although the CaKre9 protein expresses very low concentration levels, it is capable of inducing a significant increase of tumor adhesion at concentrations lower than 10 ng/ml. Nevertheless, the effect decreases when the concentrations of this protein are higher than that concentration.

Given that said recombinant CaKre9 protein has been obtained in the form of a fusion protein [CaKre9-(His)$_6$] and expressed in *E. coli*, it lacks mannosylation, which is characteristic when it is expressed in its native form in *C. albicans* and would not be able to stimulate the mannose receptors. However, this protein is capable of triggering an inflammatory response which induces a spectacular increase of tumor adhesion to the endothelium, inducing close to 100% binding of the tumor cells used when the endothelium is stimulated with a concentration of 5 ng/ml of recombinant CaKre9 protein. This result involves an increase of tumor cells adhered to the endothelium of more than 150% with respect to the control situation. This assay seems to confirm the existence of an alternative route to the one initiated by the mannose receptor which is activated in response to *C. albicans* proteins. In this case the route is possibly through the IL-1β receptor.

The existence of an immune response in the hepatic sinusoidal endothelium (HSE) against *C. albicans,* an alternative to the immune response triggered from binding to the mannose receptor, significantly but not completely reduces the increase of tumor adhesion induced by *C. albicans* and the mannoproteins thereof, together with the capacity of the recombinant CaKre9 protein to increase tumor adhesion to the endothelium demonstrate the existence of another route. In the model herein studied, said alternative route can be an inflammatory response of the endothelium against foreign antigens such as *C. albicans* antigens since the HSE cells have the capacity to function as an antigen presenting cells. In this sense, it should be noted that the *C. albicans* KRE9 mannoprotein has not been described previously as an antigen. Furthermore, it must be taken into account that said CaKre9 mannoprotein binds to the anti-murine IL-1β antibody and, therefore, can directly stimulate the IL-1β receptor of the endothelium, thus initiating the inflammatory response at that point or reinforcing the one initiated by other mannoproteins on the mannose receptor or other receptors. In addition, it must be considered that tumor adhesion on the endothelium stimulated by CaKre9 is very high at low concentrations of said protein, whereas as its concentration increases the effect decreases. A similar effect is seen in the cytokines since their autoregulatory capacity prevents them from maintaining their activity at high concentrations so as to not damage tissues. In the case of CaKre9, the inventors have confirmed that said protein is expressed in *C. albicans* in very low concentrations since said mannoprotein was only detected by means of immunodetection by using the anti-IL-1β antibody and developed with chemiluminescence.

The *C. albicans* Kre9 protein presumably acts by binding to the IL-1β receptor and has a high proinflammatory potential due to its capacity of binding to and activating the receptor of the IL-1β thereof, acting as if it was endogenous IL-1β, one of the most important proinflammatory cytokines in the immune response.

In summary, this example demonstrates that the CaKre9 protein induces a proinflammatory response mediated by proinflammatory cytokines (e.g., IL-1β, etc.) in the HSE which favors the adhesion of tumor cells to said endothelium. IL-1β is a cytokine that is crucial in said process and the blocking thereof completely inhibits tumor adhesion. This example shows that the recombinant CaKre9 protein is recognized as an antibody which recognizes and blocks IL-1β; likewise, it illustrates that the recombinant CaKre9 protein significantly stimulates tumor adhesion to the HSE at very low concentrations.

Although the mannose receptor is involved in activating a large part of the proinflammatory response of the endothelium against *C. albicans* triggering an increase of the tumor adhesion, the endothelium is also stimulated with recombinant proteins lacking mannose (e.g., recombinant CaKre9), which demonstrates the existence of other stimulation routes.

In conclusion, the hematogenous spread of *C. albicans* or the mannoproteins thereof can increase the risk of suffering tumor cells implantation in the hepatic endothelium through a proinflammatory reaction. This reaction is mostly caused by the stimulation of the mannose receptor, but the *C. albicans* effectors, such as the KRE9 mannoprotein, which act on other endothelial receptors, also intervene. Therefore, it is possible to state that the cells of *C. albicans* or some of the mannoproteins thereof would favor tumor implantation or metastatic process in the liver in the presence of tumor cells.

**Claims**

**1.** A non-glycosylated protein comprising the amino acid sequence shown in SEQ ID NO: 1, or a functionally equivalent variant or fragment thereof, capable of increasing the adhesion of tumor cells to the endothelium.

2. Protein according to claim 1, wherein said protein is the non-glycosylated Kre9 protein of a species of the genus candida (*Candida* spp.).

3. Prokaryotic organism comprising a polynucleotide encoding a non-glycosylated protein comprising the amino acid sequence shown in SEQ ID NO: 1, or a functionally equivalent variant or fragment thereof, capable of increasing the adhesion of tumor cells to the endothelium, according to claim 1.

4. Organism according to claim 3 comprising the nucleotide sequence shown in SEQ ID NO: 2.

5. Organism according to claim 3 or 4, wherein said organism is a bacterium.

6. Organism according to claim 5, wherein said organism is *Escherichia coli.*

7. A process for obtaining a non-glycosylated protein comprising the amino acid sequence shown in SEQ ID NO: 1, or a functionally equivalent variant or fragment thereof, capable of increasing the adhesion of tumor cells to the endothelium, according to claim 1, which comprises cultivating a prokaryotic organism according to any of the claims 3 to 6 under conditions which allow the production of said protein, variant or fragment, and, if desired, recovering said non-glycosylated protein, variant or fragment thereof.

8. An in vitro method for identifying a compound potentially useful for the prevention and/or treatment of a disease associated with unwanted cell adhesion, which comprises:

a) contacting a cell which expresses a receptor to which or with which a non-glycosylated protein comprising the amino acid sequence shown in SEQ ID NO: 1, or a functionally equivalent variant or fragment thereof, capable of increasing the adhesion of tumor cells to the endothelium, according to claim 1, binds or interacts with a non-glycosylated protein comprising the amino acid sequence shown in SEQ ID NO: 1, or a functionally equivalent variant or fragment thereof, capable of increasing the adhesion of tumor cells to the endothelium, according to claim 1, and with the candidate compound, and
b) identifying those compounds which inhibit the binding of said non-glycosylated protein to said receptor.

9. Use of an inhibitory agent of the activity of a non-glycosylated protein comprising the amino acid sequence shown in SEQ ID NO: 1, or a functionally equivalent variant or fragment thereof, capable of increasing the adhesion of tumor cells to the endothelium, according to claim 1, in the manufacture of a pharmaceutical composition for the prevention and/or treatment of a disease associated with unwanted cell adhesion.

10. Use according to claim 9, wherein said disease associated with unwanted cell adhesion comprises cancer, metastasis, restenosis, a disease which stimulates a cellular immune response or an inflammatory disease.

11. Use according to claim 9, wherein said inhibitory agent is an antibody which recognizes said non-glycosylated protein comprising the amino acid sequence shown in SEQ ID NO: 1, or a functionally equivalent variant or fragment thereof, capable of increasing the adhesion of tumor cells to the endothelium, according to claim 1.

12. A pharmaceutical composition comprising a therapeutically effective amount of an inhibitory agent of the activity of a non-glycosylated protein comprising the amino acid sequence shown in SEQ ID NO: 1, or a functionally equivalent variant or fragment thereof, capable of increasing the adhesion of tumor cells to the endothelium, according to claim 1, together with one or more pharmaceutically acceptable carriers.

13. Pharmaceutical composition according to claim 1, further comprising an additional drug useful in the treatment of a disease associated with unwanted cell proliferation and/or adhesion.

14. Use of an inhibitory agent of the activity of a non-glycosylated protein comprising the amino acid sequence shown in SEQ ID NO: 1, or a functionally equivalent variant or fragment thereof, capable of increasing the adhesion of tumor cells to the endothelium, according to claim 1, in the manufacture of a pharmaceutical composition for the prevention and/or treatment of a disease associated with unwanted cell adhesion.

15. Use according to claim 9, wherein said disease associated with unwanted cell adhesion, comprises cancer, metastasis, restenosis, a disease which stimulates a cellular immune response or an inflammatory disease.

16. Use according to claim 9, wherein said inhibitory agent is an antibody which recognizes said non-glycosylated protein comprising the amino acid sequence shown in SEQ ID NO: 1, or a functionally equivalent variant or fragment thereof, capable of increasing the adhesion of tumor cells to the endothelium, according to claim 1.

17. A pharmaceutical composition comprising a therapeutically effective amount of an inhibitory agent of the activity of a non-glycosylated protein comprising the amino acid sequence shown in SEQ ID NO: 1, or a functionally equivalent variant or fragment thereof, capable of increasing the adhesion of tumor cells to the endothelium, according to claim 1, together with one or more pharmaceutically acceptable carriers.

18. Pharmaceutical composition according to claim 17, further comprising an additional drug useful in the treatment of a disease associated with unwanted cell proliferation and/or adhesion.

19. Use of a non-glycosylated protein comprising the amino acid sequence shown in SEQ ID NO: 1, or a functionally equivalent variant or fragment thereof, capable of increasing the adhesion of tumor cells to the endothelium, according to claim 1, in the manufacture of a pharmaceutical composition for the prevention and/or treatment of a disease caused by an infectious agent.

20. A pharmaceutical composition comprising a therapeutically effective amount of a non-glycosylated protein comprising the amino acid sequence shown in SEQ ID NO: 1, or a functionally equivalent variant or fragment thereof, capable of increasing the adhesion of tumor cells to the endothelium, according to claim 1, together with one or more pharmaceutically acceptable carriers.

21. Pharmaceutical composition according to claim 1, further comprising an additional drug useful in the treatment of a disease caused by an infectious agent.

22. Use of a kit comprising:

an inhibitory agent of the activity of a non-glycosylated protein comprising the amino acid sequence shown in SEQ ID NO: 1, or a functionally equivalent variant or fragment thereof, capable of increasing the adhesion of tumor cells to the endothelium, according to claim 1, in the manufacture of a pharmaceutical composition for the prevention and/or treatment of a disease associated with unwanted cell adhesion;
or, alternatively,
a non-glycosylated protein comprising the amino acid sequence shown in SEQ ID NO: 1, or a functionally equivalent variant or fragment thereof, capable of increasing the adhesion of tumor cells to the endothelium, according to claim 1, in the manufacture of a pharmaceutical composition for the prevention and/or treatment of a disease caused by an infectious agent.

23. An in vitro method for identifying a compound potentially useful for the prevention and/or treatment of a disease associated with unwanted cell adhesion, which comprises:

a) Contacting a cell which expresses a receptor to which or with which a glycosylated protein comprising the amino acid sequence shown in SEQ ID NO: 1, or a functionally equivalent variant or fragment thereof, capable of increasing the adhesion of tumor cells to the endothelium binds or interacts with glycosylated protein comprising the amino acid sequence shown in SEQ ID NO: 1, or a functionally equivalent variant or fragment thereof, capable of increasing the adhesion of tumor cells to the endothelium, and with the candidate compound, and
b) identifying those compounds which inhibit the binding of said glycosylated protein to said receptor.

24. Use of an inhibitory agent of the activity of a glycosylated protein comprising the amino acid sequence shown in SEQ ID NO: 1, or a functionally equivalent variant or fragment thereof, capable of increasing the adhesion of tumor cells to the endothelium, in the manufacture of a pharmaceutical composition for the prevention and/or treatment of a disease associated with unwanted cell adhesion.

25. Use according to claim 24, wherein said disease associated with unwanted cell adhesion comprises cancer, metastasis, restenosis, a disease which stimulates a cellular immune response or an inflammatory disease.

26. Use according to claim 24, wherein said inhibitory agent is an antibody which recognizes said glycosylated protein comprising the amino acid sequence shown in SEQ ID NO: 1, or a functionally equivalent variant or fragment thereof, capable of increasing the adhesion of tumor cells to the endothelium.

27. A pharmaceutical composition comprising a therapeutically effective amount of an inhibitory agent of the activity of a glycosylated protein comprising the amino acid sequence shown in SEQ ID NO: 1, or a functionally equivalent variant or fragment thereof, capable of increasing the adhesion of tumor cells to the endothelium, together with one or more pharmaceutically acceptable carriers.

28. Pharmaceutical composition according to claim 27, further comprising an additional drug useful in the treatment of a disease associated with unwanted cell proliferation and/or adhesion.

29. Use of an inhibitory agent of the activity of a glycosylated protein comprising the amino acid sequence shown in SEQ ID NO: 1, or a functionally equivalent variant or fragment thereof, capable of increasing the adhesion of tumor cells to the endothelium, in the manufacture of a pharmaceutical composition for the prevention and/or treatment of a disease associated with unwanted cell adhesion.

30. Use according to claim 29, wherein said disease associated with unwanted cell adhesion comprises cancer, metastasis, restenosis, a disease which stimulates a cellular immune response or an inflammatory disease.

31. Use according to claim 29, wherein said inhibitory agent is an antibody which recognizes said glycosylated protein comprising the amino acid sequence shown in SEQ ID NO: 1, or a functionally equivalent variant or fragment thereof, capable of increasing the adhesion of tumor cells to the endothelium.

32. A pharmaceutical composition comprising a therapeutically effective amount of an inhibitory agent of the activity of a glycosylated protein comprising the amino acid sequence shown in SEQ ID NO: 1, or a functionally equivalent variant or fragment thereof, capable of increasing the adhesion of tumor cells to the endothelium, together with one or more pharmaceutically acceptable carriers.

33. Pharmaceutical composition according to claim 32, further comprising an additional drug useful in the treatment of a disease associated with unwanted cell proliferation and/or adhesion.

34. Use of a glycosylated protein comprising the amino acid sequence shown in SEQ ID NO: 1, or a functionally equivalent variant or fragment thereof, capable of increasing the adhesion of tumor cells to the endothelium, in the manufacture of a pharmaceutical composition for the prevention and/or treatment of a disease caused by an infectious agent.

35. A pharmaceutical composition comprising a therapeutically effective amount of a non-glycosylated protein comprising the amino acid sequence shown in SEQ ID NO: 1, or a functionally equivalent variant or fragment thereof, capable of increasing the adhesion of tumor cells to the endothelium, together with one or more pharmaceutically acceptable carriers.

36. Pharmaceutical composition according to claim 1, further comprising an additional drug useful in the treatment of a disease caused by an infectious agent.

37. Use of a kit comprising:

an inhibitory agent of the activity of a glycosylated protein comprising the amino acid sequence shown in SEQ ID NO: 1, or a functionally equivalent variant or fragment thereof, capable of increasing the adhesion of tumor cells to the endothelium, in the manufacture of a pharmaceutical composition for the prevention and/or treatment of a disease associated with unwanted cell adhesion; or, alternatively, a glycosylated protein comprising the amino acid sequence shown in SEQ ID NO: 1, or a functionally equivalent variant or fragment thereof, capable of increasing the adhesion of tumor cells to the endothelium, in the manufacture of a pharmaceutical composition for the prevention and/or treatment of a disease caused by an infectious agent.

Figure 1

Figure 2

Figure 3

TATTTGCTTTAAAGATATCGGATACTTTTTTTTCCTTTTGAGTTTTGTTTTGTTTTGTTTTATT
TAGATTAGTTTCTATTTTTAATTAATATTCTACATTCGTTATTGACGACTACAACATATCAACG
ACCTAGCACATCCACCCACCAAGATAACACAACTACAAT**CTATCAGACACATCTAGCAAA**_ATGA_
_GACAATTTCAAATCATATTAATTTCCCTTGTTGTTTCCATAATAAGATGTGTTGTTGCAGATGT_
_TGACATCACATCACCAAAGAGTGGAGAAACTTTTTCTGGTAGTTCTGGATCAGCAAGTATCAAG_
_ATTACCTGGGATGATTCAGACGATTCAGACTCACCGAAATCTTTGGATAATGCCAAAGGGTACA_
_CAATTTCTTTATGTACTGGACCTACTTCAGATGGGGATATCCAGTGTTTGGATCCATTAGTCAA_
_GAACGAAGCTATTTCAGGTAAATCTAAAACAGTTTCCATTGCCCAGAACTCAGTACCTAATGGT_
_TATTACTATTTCCAAATTTACGTTACTTTCACTAATGGAGGTACCACTATTCATTATTCACCAC_
_GTTTCAAATTGACTGGTATGTCTGGTCCAACTGCCACTTTAGATGTCACCGAAACAGGATCAGT_
_GCCAGCGGATCAAGCTTCAGGATTTGATACTGCAACTACTGCTGACTCCAAATCTTTCACAGTT_
_CCATATACCCTACAAACAGGGAAGACTAGATACGCACCAATGCAAATGCAACCAGGTACCAAAG_
_TGACTGCTACAACCTGGAGTATGAAGTTCCCAACTAGTGCTGTTACTTACTACTCAACAAAGGC_
_TGGCACACCAAATGTGGCCTCTACTATTACCCCAGGTTGGAGTTATACTGCTGAATCTGCCGTT_
_AACTATGCTAGTGTTGCGCCATATCCAACATACTGGTATCCTGCCAGTGAACGAGTGAGTAAGG_
_CTACAATTAGTGCTGCTACAAAG_AGAAGAAGATGGTTGGAT_TGA_ATGAGGGAAACGGTGCTTAT
AACTTAGATAATGCTAACATTGGATTTCATTTTTCATTTTTTTTTGTTTGGTTTAGTTTGTTTA
TTAGCTTGTTCAATAACATACAGATCCAAGCTCAGCTCAAGACACAAGCAAAAAGAC

Figure 4

# INTERNATIONAL SEARCH REPORT

| | |
|---|---|
| International application No. | |
| PCT/ ES 2010/070196 | |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

C07K, A61K, A61P

Documentation searched other than minimum documentation to the extent that such documents are included in the fields searched

Electronic data base consulted during the international search (name of data base and, where practicable, search terms used)

INVENES, EPODOC, WPI, TXTE, XPESP, BIOSIS, MEDLINE, NPL, EMBASE, EBI

**C. DOCUMENTS CONSIDERED TO BE RELEVANT**

| Category* | Citation of document, with indication, where appropriate, of the relevant passages | Relevant to claim No. |
|---|---|---|
| X | 26.04.2005, Base of datos EBI [online] [retrieved on 13-05-2010] Retrieved from : http://srs.ebi.ac.uk/srsbin/cgi-bin/wgetz?-e+[uniprot-id:Q5ANN9_CANAL]\|[uniprot-acc:Q5ANN9_CANAL] | 1-7, 12, 13, 20, 21, 27, 28 |
| X | US 7241613 B1 (WILLINS et al. 10.07.2007) 10.07.2007, (the whole document). | 1-7, 12, 13, 19-21, 27,28, 34 |
| X | Lussier M. et al. The *Candida albicans* KRE9 gene is required for cell wall β-1,6-glucan synthesis and is essential for growth on glucose. Proc. Natl. Acad. Sci. USA. 08.1998, Vol. 95, pages 9825-9830 (the whole document). | 1-7, 12, 13, 20, 21, 27, 28 |
| X | WO 99/31269 A2 (MCGILL UNIVERSITY) 24.06.1999, (the whole document). | 1-7, 12, 13, 20, 21, 27, 28 |

☒ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
|---|---|
| 25 May 2010　　　(25.05.2010) | **(09/06/2010)** |
| Name and mailing address of the ISA/ O.E.P.M. Paseo de la Castellana, 75 28071 Madrid, España. Facsimile No.　34 91 3495304 | Authorized officer M. Cumbreño Galindo Telephone No. +34 91 349 68 80 |

Form PCT/ISA/210 (second sheet) (July 2009)

INTERNATIONAL SEARCH REPORT

| International application No. |
|---|
| PCT/ ES 2010/070196 |

**Box No. II** Observations where certain claims were found unsearchable (Continuation of item 2 of first sheet)

This international search report has not been established in respect of certain claims under Article 17(2)(a) for the following reasons:

1. ☐ Claims Nos.:
because they relate to subject matter not required to be searched by this Authority, namely:

2. ☒ Claims Nos.:
because they relate to parts of the international application that do not comply with the prescribed requirements to such an extent that no meaningful international search can be carried out, specifically:

See extra sheet

3. ☐ Claims Nos.:
because they are dependent claims and are not drafted in accordance with the second and third sentences of Rule 6.4(a).

**Box No. III** Observations where unity of invention is lacking (Continuation of item 3 of first sheet)

This International Searching Authority found multiple inventions in this international application, as follows:

1. ☐ As all required additional search fees were timely paid by the applicant, this international search report covers all searchable claims.

2. ☒ As all searchable claims could be searched without effort justifying additional fees, this Authority did not invite payment of additional fees.

See extra sheet

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (July 2009)

## INTERNATIONAL SEARCH REPORT

International application No.

PCT/ES 2010/070196

C (continuation).                           DOCUMENTS CONSIDERED TO BE   RELEVANT

| Category* | Citation of documents, with indication, where appropriate, of the relevant passages | Relevant to claim No. |
|---|---|---|
| X | WO 02/053728 A2 (ELITRA PHARMACEUTICALS INC. 11.07.2002 ) 11.07.2002, (the whole document). | 1-7, 12, 13, 19-21, 27, 28, 34 |
| A | Orozco A. S. et al. Mechanisms of the proinflammatory response of indotelial cells to *Candida albicans* infection. Infection and Immunity. 03.2000, Vol. 68, Nº3, pages 1134-1141, ISSN 0019-9567/00 (the whole document) | 1-13, 19-21, 23-28, 34 |
| A | Polonelli L. et al. Antibody Complementarity-Determining Regions (CDRs) can display differential antimicrobial, antiviral and antitumor activities. Plos ONE. 11.06.2008, Vol. 3, Nº 6, e2371 (the whole document) | 1-13, 19-21, 23-28, 34 |

Form PCT/ISA/210 (continuation of second sheet) (July 2009)

## INTERNATIONAL SEARCH REPORT
Information on patent family members

International application No.

PCT/ ES 2010/070196

| Patent document cited in the search report | Publication date | Patent family member(s) | Publication date |
|---|---|---|---|
| US 7241613 B | 10.07.2007 | NONE | ------------ |
| WO 9931269 A | 24.06.1999 | CA 2218446 A<br>CA 2314611 A<br>EP 1036200 A<br>EP 19980960963<br>US 6582911 B | 12.06.1999<br>24.06.1999<br>20.09.2000<br>10.12.1998<br>24.06.2003 |
| WO 02053728 A | 11.07.2002 | CA 2432902 A<br>US 2003180953 A<br>EP 1348027 A<br>EP 20010991419<br>US 6783985 B<br>US 2005079619 A<br>JP 2005514899 T | 11.07.2002<br>25.09.2003<br>01.10.2003<br>26.12.2001<br>31.08.2004<br>14.04.2005<br>26.05.2005 |

Form PCT/ISA/210 (patent family annex) (July 2009)

**INTERNATIONAL SEARCH REPORT**

International application No.

PCT/ ES 2010/070196

CLASSIFICATION OF SUBJECT MATTER

*C07K 14/395* (2006.01)
*A61K 36/064* (2006.01)
*A61K 39/395* (2006.01)
*A61P 31/10* (2006.01)
*A61P 35/04* (2006.01)

Form PCT/ISA/210 (extra sheeet) (July 2009)

| INTERNATIONAL SEARCH REPORT | International application No. |
|---|---|
| | PCT/ ES 2010/070196 |

Box II.2.

Claims 14-18, 22, 29-33, 35, 36 and 37

Claims 14-18, 29-33, 35 and 36 and claims 22 and 37, insofar as the latter relate to the use of the protein, are redundant, that is are repeated. In addition, claims 22 and 37, insofar as they relate to the use of the kit, are unclear.

Box III

The application does not meet the requirement of unity of invention, since it does not contain a single invention or group of inventions so linked as to form a single general inventive concept (PCT Rule 13.1). The International Searching Authority is of the opinion that the application contains two inventions, covered by the following claims:

INVENTION 1

The present invention relates to:

- a non-glycosylated protein comprising the sequence SEQ ID NO: 1, or a functionally equivalent variant or fragment thereof, capable of increasing the adhesion of tumour cells to the endothelium, said protein being the protein Kre9 of Candida spp (claims 1 and 2), a procaryotic organism comprising a polynucleotide that codes for said protein (claims 3 to 6) and the method for obtaining the protein using said procaryotic organism (claim 7);

- an in vitro method for identifying a compound that may be useful in the prevention and/or treatment of a disease associated with undesirable cellular adhesion, involving contacting a cell that expresses a receptor to which bonds a non-glycosylated protein comprising the sequence SEQ ID NO: 1, or a functionally equivalent variant or fragment thereof, with said protein and with the candidate compound and identifying the compounds which inhibit the bonding of the protein to the receptor (claim 8);

- the use of an antibody that recognises and inhibits the activity of a non-glycosylated protein comprising the sequence SEQ ID NO: 1, or a functionally equivalent variant or fragment thereof, in preparing a pharmaceutical composition for the prevention and/or treatment of a disease associated with undesirable cellular adhesion (claims 9 to 11);

- a pharmaceutical composition which comprises a therapeutically effective quantity of an antibody that inhibits the activity of the non-glycosylated protein comprising the sequence SEQ ID NO: 1, or a functionally equivalent variant or fragment thereof, and which may also comprise an additional drug that is useful in the treatment of a disease associated with undesirable cellular adhesion (claims 12 and 13);

- a pharmaceutical composition which comprises a therapeutically effective quantity of a non-glycosylated protein comprising the sequence SEQ ID NO: 1, or a functionally equivalent variant or fragment thereof, and which also comprises an additional drug that is useful in the treatment of a disease caused by an infectious agent (claims 20 and 21);

- an in vitro method for identifying a compound that may be useful in the prevention and/or treatment of a disease associated with undesirable cellular adhesion, involving contacting a cell that expresses a receptor to which bonds a glycosylated protein comprising the sequence SEQ ID NO: 1, or a functionally equivalent variant or fragment thereof, with said protein and with the candidate compound and identifying the compounds which inhibit the bonding of said protein to the receptor (claim 23);

- the use of an antibody that recognises and inhibits the activity of a glycosylated protein comprising the sequence SEQ ID NO: 1, or a functionally equivalent variant or fragment thereof, in preparing a pharmaceutical composition for the prevention and/or treatment of a disease associated with undesirable cellular adhesion (claims 24 to 26);

- a pharmaceutical composition which comprises a therapeutically effective quantity of an antibody that inhibits the activity of the glycosylated protein comprising the sequence SEQ ID NO: 1, or a functionally equivalent variant or fragment thereof, and which also comprises an additional drug that is useful in the treatment of a disease associated with undesirable cellular adhesion (claims 27 and 28).

INVENTION 2

- the use of a non-glycosylated protein comprising the sequence SEQ ID NO: 1, or a functionally equivalent variant or fragment thereof, in preparing a pharmaceutical composition for the prevention and/or treatment of a disease caused by an infectious agent (claim 19);

- the use of a glycosylated protein comprising the sequence SEQ ID NO: 1, or a functionally equivalent variant or fragment thereof, in preparing a pharmaceutical composition for the prevention and/or treatment of a disease caused by an infectious agent (claim 34).

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6582911 B1
- US 6582911 B

### Non-patent literature cited in the description

- **Lussier et al.** *Proc Natl Acad Sci.,* 1998, vol. 95, 9825-9830
- **Altschul et al.** *Nucleic Acids Res.,* 1997, vol. 25, 3389
- **Sambrook et al.** Molecular cloning, a Laboratory Manual. Cold Spring Harbor Laboratory Press, 1989, vol. 1-3
- **Studier ; Moffatt.** *J. Mol. Biol.,* 1986, vol. 189, 113-130
- **Köhler ; Milstein.** *Nature,* 1975, vol. 256, 495-397
- **Harlow ; Lane.** Using Antibodies. A Laboratory Manual. Cold Spring Harbor Laboratory Press, 1998
- Handbook of Therapeutic Antibodies. Wiley-VCH, 2007, vol. I-III
- Antibodies: Volume 1: Production and Purification. Springer, 2004, vol. 1
- Antibodies: Volume 2: Novel Technologies and Therapeutic Use. Springer, 2004
- Molecular Cloning: a Laboratory Manual. Cold Spring Harbor Laboratory Press, 2001
- Merck Index in CD-ROM
- **C. Faulí i Trillo.** Tratado de Farmacia Galénica. 1993
- **C. Faulí ; Trillo.** Tratado de Farmacia Galénica. 1993
- **Le Gouill, C. ; Déry, C.V.** *Nucleic Acids Research.,* 1991, vol. 19 (23), 6655
- **Fidler IJ.** *Nature.,* 1973, vol. 242, 148-149